# EUROPÄISCHE PATENTSCHRIFT

(11) **EP 1 446 224 B1**
(45) Veröffentlichungstag und Bekanntmachung des Hinweises auf die Patenterteilung: **25.01.2006**
(21) Anmeldenummer: 02803396.7
(22) Anmeldetag: 21.11.2002
(51) Int. Cl.: B01J 31/18, C07F 9/46, C07F 9/547, C07F 9/58, C07F 9/6512, C07F 9/60, C07F 9/6558, C07F 15/00, C07F 15/04, C07B 37/04, C07B 41/00, C07B 43/04, C07D 213/76, C07D 239/42, C07D 241/20, C07D 215/38, C07D 401/12

(54) **P-FUNKTIONALISIERTE AMINE N-HALTIGER AROMATEN, DEREN HERSTELLUNG UND IHRE VERWENDUNG IN KATALYTISCHEN REAKTIONEN**
AROMATIC COMPOUNDS CONTAINING NITROGEN AND P-FUNCTIONALIZED AMINES, THE PRODUCTION THEREOF AND THEIR USE IN CATALYTIC REACTIONS
PRODUITS AROMATIQUES AZOTES RENFERMANT DES AMINES FONCTIONNALISEES P, LEUR PRODUCTION ET LEUR UTILISATION DANS DES REACTIONS CATALYTIQUES

(30) Priorität: 23.11.2001 DE 10157358
(43) Veröffentlichungstag der Anmeldung: 18.08.2004
(73) Patentinhaber: Degussa AG, 40474 Düsseldorf (DE)
(72) Erfinder: KEMPE, Rhett, 13189 Berlin (DE); SCHAREINA, Thomas, 18195 Cammin (DE); MONSEES, Axel, 60487 Frankfurt (DE); RIERMEIER, Thomas, 65439 Flörsheim (DE)
(86) Internationale Anmeldenummer: PCT/EP2002/013048
(87) Internationale Veröffentlichungsnummer: WO 2003/043735

(56) Entgegenhaltungen:
- EP-A- 0 124 154
- EP-A- 1 132 361
- US-A- 5 977 361
- SCHAREINA, T. ET AL.: "Combinatorial Libraries with P-Functionalized Aminopyridines: Ligands for the Preparation of Efficient C(Aryl)-Cl Activation Catalysts" ANGEWANDTE CHEMIE, INTERNATIONAL EDITION ENGLISH, Bd. 41, Nr. 9, 2. Mai 2002 (2002-05-02), Seiten 1521-1523, XP002245409
- DATABASE CA [Online] CHEMICAL ABSTRACTS SERVICE, COLUMBUS, OHIO, US; WRACKMEYER, BERND ET AL: "Aminophosphanes with bulky amino groups: molecular structure, coupling constants 1J(31P, 15N) and 2J(31P, 29Si), and isotope-induced chemical shifts 1.DELTA.14/15N(31P)" retrieved from STN Database accession no. 138:271743 XP002245416 & HETEROATOM CHEMISTRY (2002), 13(7), 667-676 ,
- BUCHMEISER M R ET AL: "Bis(pyrimidine)-based palladium catalysts: synthesis, X-ray structure and applications in Heck-, Suzuki-, Sonogashira-Hagihara couplings and amination reactions" JOURNAL OF ORGANOMETALLIC CHEMISTRY, ELSEVIER-SEQUOIA S.A. LAUSANNE, CH, Bd. 634, Nr. 1, 13. September 2001 (2001-09-13), Seiten 39-46, XP004306461 ISSN: 0022-328X in der Anmeldung erwähnt
- SILBERG J ET AL: "N-Acyl-N,N-dipyridyl and N-acyl-N-pyridyl-N-quinoyl amine based palladium complexes. Synthesis, X-ray structures, heterogenization and use in Heck couplings" JOURNAL OF ORGANOMETALLIC CHEMISTRY, ELSEVIER-SEQUOIA S.A. LAUSANNE, CH, Bd. 622, Nr. 1-2, 9. März 2001 (2001-03-09), Seiten 6-18, XP004230323 ISSN: 0022-328X in der Anmeldung erwähnt
- WAGAW, SEBLE ET AL: "The Synthesis of Aminopyridines: A Method Employing Palladium-Catalyzed Carbon-Nitrogen Bond Formation" JOURNAL OF ORGANIC CHEMISTRY (1996), 61(21), 7240-7241 , XP002245410 in der Anmeldung erwähnt
- SCHAREINA, T. ET AL.: "Dipyridylamine Ligands - Synthesis, Coordination Chemistry of the Group 10 Metals and Application of Nickel Complexes in Ethylene Oligomerization" EUROPEAN JOURNAL OF INORGANIC CHEMISTRY, Nr. 9, 30. Juli 2001 (2001-07-30), Seiten 2421-2426, XP002245411 in der Anmeldung erwähnt
- LITTKE, ADAM F.; FU, GREGORY C. : "Palladium-Catalyzed Coupling Reactions of Aryl Chlorides" ANGEWANDTE CHEMIE INTERNATIONAL EDITION, Bd. 41, Nr. 22, 12. November 2002 (2002-11-12), Seiten 4176-4211, XP002245412
- PERIS, E. ET AL.: "A Pd complex of a tridentate pincer CNC bis-carbene ligand as a robust homogenous Heck catalyst" CHEMICAL COMMUNICATIONS, Nr. 2, Januar 2001 (2001-01), Seiten 201-202, XP002245413
- WOLFE J P: "Highly active palladium catalysts for Suzuki coupling reactions" JOURNAL OF THE AMERICAN CHEMICAL SOCIETY, AMERICAN CHEMICAL SOCIETY, WASHINGTON, DC, US, Bd. 121, Nr. 41, 20. Oktober 1999 (1999-10-20), Seiten 9550-9561, XP002132767 ISSN: 0002-7863
- HASSAN, J.; LEMAIRE, M. ET AL.: "Aryl-Aryl Bond Formation One Century after the Discovery of the Ullmann Reaction" CHEMICAL REVIEWS, Bd. 102, Nr. 5, 8. März 2002 (2002-03-08), Seiten 1359-1469, XP002245414
- YANG, H. ET AL.: "Ruthenium(II) Complexes Containing Optically Active Hemilabile P,N,O-Tridentate Ligands. Synthesis and Evaluation in Catalytic Asymmetric Transfer Hydrogenation of Acetophenone by Propan-2-ol" ORGANOMETALLICS, Bd. 16, Nr. 7, 1997, Seiten 1401-1409, XP002245415
- WRACKMEYER BERND ET AL: 'Aminophosphanes with bulky amino groups: molecular structure, coupling constants 1J(31P, 15N) and 2J(31P, 29Si), and isotope-induced chemical shifts 1.DELTA.14/15N(31P)' HETEROATOM CHEMISTRY Bd. 13, Nr. 7, 05 November 2002, Seiten 667 - 676, XP009012805
- KOTHA S. ET AL: 'Recent applications of the Suzuki-Miyaura cross-coupling reaction in organic synthesis' TETRAHEDRON, ELSEVIER SCIENCE PUBLISHERS, AMSTERDAM, NL Bd. 58, Nr. 48, 13 November 2002, Seiten 9633 - 9695, XP004393976
- LITTKE A.F. ET AL: 'Palladiumkatalysierte Kupplungen von Arylchloriden' ANGEWANDTE CHEMIE Bd. 114, Nr. 22, 12 November 2002, Seiten 4350 - 4386
- ZAPF A. ET AL: 'Palladium/Phosphite Catalyst Systems for Efficient Cross Coupling of Aryl Bromides and Chlorides with Phenylboronic Acid' CHEMISTRY - A EUROPEAN JOURNAL Bd. 6, Nr. 10, 15 Mai 2000, Seiten 1830 - 1833
- LI G.Y.: 'The First Phosphine Oxide Ligand Precursors for Transition Metal Catalyzed Cross-Coupling Reactions: C-C, C-N, and C-S Bond Formation on Unactivated Aryl Chlorides' ANGEWANDTE CHEMIE Bd. 113, Nr. 8, 17 April 2001, Seiten 1561 - 1564

## Beschreibung

Die vorliegende Erfindung betrifft neue Liganden für Übergangsmetalle, deren Herstellung und ihre Verwendung in katalytischen Reaktionen, besonders zur Veredelung von Halogenaromaten.

Halogenaromaten, darunter insbesondere Chloraromaten sind vielfältig nutzbare Zwischenprodukte der chemischen Industrie, die als Vorprodukte für die Herstellung von Agrointermediaten, Pharmazeutika, Farbstoffen etc. dienen. Auch Vinylhalogenide sind wichtige Zwischenprodukte, die als Ausgangsstoffe für Polymere und zur Herstellung der zuvor genannten Produkte verwendet werden.

Häufig angewandte Katalysatoren zur Funktionalisierung von Halogenaromaten oder Vinylhalogeniden zu aromatischen Olefinen bzw. Dienen (Heck-Reaktion, Stille-Reaktion), Biarylen (Suzuki-Reaktion), Alkinen (Sonogashira-Reaktion), Carbonsäurederivaten (Heck-Carbonylierung), Aminen (Buchwald-Hartwig-Reaktion) sind Palladium- und Nickelkatalysatoren. Dabei sind Palladiumkatalysatoren generell vorteilhaft, was die Breite der Anwendbarkeit von Kupplungssubstraten und teilweise die Katalysatoraktivität angeht, während Nickelkatalysatoren Vorteile im Bereich der Umsetzung von Chloraromaten und Vinylchloriden und in Bezug auf den Metallpreis besitzen.

Palladium- und Nickelkatalysatoren, die im Rahmen der Aktivierung und weiteren Veredelung von Halogenaromaten verwendet werden, sind sowohl Palladium(II)-und/oder Nickel(II)- als auch Palladium(0)- und/oder Nickel(0)-Komplexe, obwohl es bekannt ist, daß Palladium(0)- bzw. Nickel(0)-Verbindungen die eigentlichen Katalysatoren der Reaktion sind. Insbesondere formuliert man gemäß Angaben in der Literatur koordinativ ungesättigte 14- und 16-Elektronen Palladium(0)- bzw.Nickel(0)-Komplexe, welche mit Donorliganden wie Phosphanen stabilisiert werden, als aktive Spezies.

Beim Einsatz von lodiden als Edukte in Kupplungsreaktionen ist es auch möglich, auf Phosphanliganden zu verzichten. Allerdings sind Aryl- und Vinyliodide sehr teure Ausgangsverbindungen, wobei darüber hinaus bei deren Umsetzung stöchiometrische Mengen an lodsalz-Abfällen anfallen. Werden kostengünstigere Edukte in der Heck-Reaktion eingesetzt, wie Arylbromide oder Arylchloride, dann ist der Zusatz von stabilisierenden und aktivierenden Liganden notwendig, damit die Edukte katalytisch effektiv umgesetzt werden können.

Die für Olefinierungen, Alkinylierungen, Carbonylierungen, Arylierungen, Aminierungen und ähnliche Reaktionen beschriebenen Katalysatorsysteme weisen häufig nur mit nicht ökonomischen Ausgangsmaterialien wie lodaromaten und aktivierten Bromaromaten befriedigende katalytische Wechselzahlen ("turnover numbers" = TON) auf. Ansonsten müssen bei deaktivierten Bromaromaten und insbesondere bei Chloraromaten generell große Mengen an Katalysator - üblicherweise mehr als 1 Mol-% - zugesetzt werden, um technisch nutzbare Ausbeuten (> 90%) zu erzielen. Aufgrund der Komplexität der Reaktionsgemische ist zudem kein einfaches Katalysatorrecycling möglich, so daß auch die Rückführung des Katalysators hohe Kosten verursacht, die in der Regel einer technischen Realisierung entgegenstehen. Darüber hinaus ist es besonders bei der Herstellung von Wirkstoffen bzw. Wirkstoffvorprodukten unerwünscht, mit großen Mengen an Katalysator zu arbeiten, da ansonsten in diesem Fall Katalysatorrückstände im Produkt verbleiben.

Neuere aktive Katalysatorsysteme basieren auf cyclopalladierten Phosphanen (EP 1 132 361; W. A. Herrmann, C. Broßmer, K. Öfele, C.-P. Reisinger, T. Priermeier, M. Beller, H. Fischer, *Angew. Chem*. **1995**, *107*, 1989; *Angew. Chem. Int. Ed. Engl.* **1995,** *34*, 1844) oder Gemischen von sterisch anspruchsvollen Arylphosphanen (J. P. Wolfe, S. L. Buchwald, *Angew. Chem.* **1999,** *111*, 2570; *Angew. Chem. Int. Ed. Engl.* **1999,** *38*, 2413) bzw. Tri-tert. butylphosphan (A. F. Littke, G. C. Fu, *Angew. Chem.* **1998,** *110*, 3586; *Angew. Chem. Int. Ed. Engl.* **1998**, *37*, 3387) mit Palladiumsalzen oder Palladiumkomplexen.

Kostengünstige Chloraromaten sind jedoch auch mit diesen Katalysatoren generell nicht technisch befriedigend zu aktivieren, d.h. Katalysatorproduktivitäten (TON) sind < 10000 und Katalysatoraktivitäten (TOF) sind < 1000 h⁻¹. Somit müssen für das Erreichen von hohen Ausbeuten vergleichsweise große Katalysatormengen eingesetzt werden, was mit hohen Kosten verbunden ist. So betragen beispielsweise die Katalysatorkosten für die Herstellung von einem Kilogramm eines organischen Zwischenprodukts mit dem Molekulargewicht 200 bei Verwendung von 1 Mol-% Palladiumkatalysator bei derzeitigen Edelmetallpreisen mehr als 100 US$, was die Notwendigkeit zur Verbesserung der Katalysatorproduktivität deutlich macht. Daher sind trotz aller Weiterentwicklungen der Katalysatoren in den letzten Jahren bis dato nur wenige industrielle Umsetzungen der Arylierung, Carbonylierung, Olefinierung etc. von Chloraromaten bekannt geworden.

Das Dokument EP-A-1 132 361 offenbart katalytisch aktive Pd-Komplexe, enthaltend sekundäre Phosphine, entsprechend dem ggw. definierten Fragment R¹R²P, als Liganden und optionale, getrennt zuzusetzende weitere N-Heterozyklische Liganden auf Pyridinbasis, welche ebenfalls in 2-Stellung Aminosubstituiert sein können, jedoch der weitere Substituent an der Aminogruppe eine C-palladierte Arylfunktion sein muß, somit ein Palladazyklus ausgebildet wird. Es werden eine Reihe von Reaktionen katalysiert. Insbesondere wird die Aktivierung von Aryl- oder Vinylchloriden hervorgehoben und in den Arbeitsbeispielen beschrieben. So werden in letzteren diverse Chloraromaten in zu Synthesen von α-Arylketonen analogen Malodinitril-Kupplungen, Suzuki-Kupplungen, Carbonylierungen zu Säuren und Estern und Heck-Reaktionen eingesetzt, mit bis zu quantitativen Ausbeuten. Insbesondere werden die deaktivierten Chloraromaten 4-Chloranisol bzw. 4-Chlortoluol für Malodinitril-Kupplungen, Suzuki-Kupplungen und Heck-Reaktionen eingesetzt, mit vorteilhaften Ausbeuten. Ziel von D1 ist die Bereitstellung von einfachen Liganden für Pd Katalysatorsysteme, welche mindestens vergleichbare Produktivität in den geschilderten Reaktionen gegenüber herkömmlichen Liganden wie z.B. tertiaren Alkylphposphanen aufweisen sollen.

Aus den genannten Gründen lag der vorliegenden Erfindung die Aufgabe zugrunde, den großen Bedarf an neuen produktiveren Katalysatorsystemen, die einfache Liganden aufweisen und die nicht die Nachteile der bekannten katalytischen Verfahren zeigen, die für die großtechnische Durchführung geeignet sind und die kostengünstige Chlor- und Bromaromaten sowie entsprechende Vinylverbindungen in hoher Ausbeute, Katalysatorproduktivität und Reinheit zu den jeweiligen Kupplungsprodukten umsetzen, zu befriedigen.

Diese Aufgabe wird erfindungsgemäß gelöst durch die Entwicklung neuer P-funktionalisierter Amine N-haltiger Aromaten, insbesondere durch P-funktionalisierte Aminopyridine, der allgemeinen Formel

R¹(R²)P-N(R')R" (I)

worin
R¹ und R² unabhängig voneinander für einen Rest stehen, ausgewählt aus der Gruppe bestehend aus C₁-C₂₄ Alkyl, C₃-C₈ Cycloalkyl und aromatische Reste mit 5 bis 14 C-Atomen, insbesondere Phenyl, Naphthyl oder Fluorenyl, wobei die Alkyl- und Cycloalkylreste

verzweigt oder unverzweigt sein können. Die cyclischen aliphatischen oder aromatischen Reste sind bevorzugt 5 oder 6 gliedrige Ringe.

Die vorgenannten Reste können selbst jeweils ein- oder mehrfach substituiert sein. Diese Substituenten können dabei unabhängig voneinander Wasserstoff, C₁-C₂₀ Alkyl, C₁-C₁₀ Haloalkyl, bevorzugt Trifluormethyl und Trichlormethyl, Halogeno, besonders Fluoro und Chloro, sein,
wobei
R¹ und R² auch miteinander verbrückt sein können, vorzugsweise unter Ausbildung eines 4-8 gliedrigen Zyklus, der gesättigt, ungesättigt oder aromatisch sein kann.

Bevorzugt handelt es sich bei R¹ und R² unabhängig voneinander um einen Rest ausgewählt aus der Gruppe bestehend aus Phenyl, Cyclohexyl, Alkyl, 2-Alkylphenyl, 3-Alkylphenyl, 4-Alkylphenyl, 2,6-Dialkylphenyl, 3,5-Dialkylphenyl, 3,4,5-Trialkylphenyl, 2-Alkoxyphenyl, 3-Alkoxyphenyl, 4-Alkoxyphenyl, 2,6-Dialkoxyphenyl, 3,5-Dialkoxyphenyl, 3,4,5-Trialkoxyphenyl, 3,5-Dialkyl-4-Alkoxyphenyl.

Besonders bevorzugt handelt es sich bei R¹ und R² unabhängig voneinander um einen Rest ausgewählt aus der Gruppe bestehend aus Phenyl, Cyclohexyl und tert. Butyl.

In einer bevorzugten Ausführungsform sind die Reste R¹ und R² identisch.

Bei R' handelt es sich um einen mindestens ein N-Atom enthaltenden aromatischen Rest mit 4 bis 13 C-Atomen, der in 2-Stellung zu dem mindestens einen aromatischen N-Atom, an das Stickstoffatom gemäß Formel I gebunden ist. Bis zu drei der genannten aromatischen C-Atome können hierbei auch durch ein weiteres Heteroatom, unabhängig voneinander ausgewählt, aus der Gruppe bestehend aus N, O und S ersetzt sein.

Bevorzugt handelt es sich bei dem aromatischen Rest mit 4 bis 13 C-Atomen um Pyrimidyl, Pyrazinyl, Pyridyl oder Chinolinyl.

Bei R" handelt es sich um Trimethylsilyl oder um einen aromatischen Rest, wobei es sich bei R" um 3-Methylpyridyl, 4-Methylpyridyl, 6-Methylpyridyl, 4,6-Dimethylpyridyl, 6-Methoxypyridyl, Pyridyl, Pyrimidyl, Pyrazinyl, 4-Methylchinolinyl, Py₂(o-P), Phenyl, Naphthyl, Fluorenyl und Trimethylsilyl handelt, wobei die N-haltigen aromatischen Reste in 2-Stellung an das N-Atom gemäß Formel I gebunden ist.

Bevorzugt handelt es sich bei dem aromatischen Rest um Pyrimidyl, Pyrazinyl, Pyridyl, Chinolinyl, Phenyl, Fluorenyl oder Naphthyl.

In einer bevorzugten Ausführungsform handelt es sich bei R" um den gleichen Rest wie bei R', wobei der Rest vorzugsweise in gleicher Weise wie R', also in 2-Position zum aromatischen N-Atom, an das N-Atom gemäß Formel (I) gebunden ist.

Die für R' und R" aufgeführten Reste können desweiteren unabhängig voneinander neben Wasserstoffatomen jeweils mindestens einen, besonders bevorzugt bis zu drei, Substituenten aufweisen, die unabhängig voneinander ausgewählt sein können aus der Gruppe bestehend aus C₁- bis C₈-Alkyl, O-Alkyl(C₁-C₈), Aryl, Fluor, NHAryl, NAryl₂,
wobei Aryl einen Aromaten mit 5 bis 14 Ringkohlenstoffatomen darstellt wobei ein oder mehrere Ringkohlenstoffatome durch Stickstoff-, Sauerstoff- und/oder Schwefelatome ersetzt sein können und die Alkylreste verzweigt, unverzweigt und/oder zyklisch sein können.

Heteroaromatische Reste können z. B. mindestens fünfgliedrige Ringe enthaltend 1 bis 13 Ringkohlenstoffatome sein, die bis zu 4 Stickstoffatome und/oder bis zu 2 Sauerstoff oder Schwefelatome enthalten. Bevorzugte heteroaromatische Arylreste enthalten ein oder zwei Stickstoff- oder ein Sauerstoff- oder ein Schwefel- oder ein Stickstoff- und ein Sauerstoff- oder Schwefelheteroatom.

Bevorzugt handelt es sich bei dem mindestens einen Substituenten für R' und R" um eine Gruppe ausgewählt aus C₁-C₈-Alkyl, O-Alkyl(C₁-C₈), Phenyl, Aryl, Fluor, CF₃.

Bei dem substituierten Rest R' handelt es sich besonders bevorzugt um einen Rest ausgewählt aus der Gruppe bestehend aus 3-Methylpyridyl, 4-Methylpyridyl, 6-Methylpyridyl, 4,6-Dimethylpyridyl, 6-Methoxypyridyl, Pyridyl, Pyrimidyl, Pyrazinyl, 4-Methylchinolinyl (s. Tab. 1), wobei R' in 2-Stellung an das N-Atom gemäß Formel (I) gebunden ist.

Ein weiterer Gegenstand der vorliegenden Erfindung ist ein Verfahren zur Herstellung der P-funktionalisierten N-haltigen aromatischen Amin-Liganden, wobei in Gegenwart einer starken Base, eine Verbindung der Formel NHR'R" mit einer Verbindung der Formel R¹(R²)PX umgesetzt wird, wobei R¹, R² , R' und R" dieselbe Bedeutung haben wie zu Formel (1) angegeben und wobei X vorzugsweise für ein Halogenatom, insbesondere für Chlor oder Brom steht. Bei der starken Base handelt es sich vorzugsweise um ein metallorganisches Reagens, besonders bevorzugt um Butyllithium, sec. Butyllithium, tert. Butyllithium oder Lithiumdiisopropylamin. Die Reaktion ist in einem organischem Lösungsmittel, beispielsweise Hexan, unter anaeroben Bedingungen durchführbar.

Amine der Formel NHR'R" und insbesondere eine Vielzahl von Bipyridylaminen, 2-Aminopyridinen oder verwandte N-heterocyclische Amine, die als Ausgangsverbindungen zur Herstellung der erfindungsgemäßen Liganden verwendet werden können, können hierbei mittels Palladium-katalysierter Arylaminierungen ausgehend von primären Aminen auf klassischem Wege nach dem folgenden Schema dargestellt werden (S. Wagaw, S.L. Buchwald, J. Org. Chem. 1996, 61, 7240; J. Stilberg et al., J. Organomet. Chem. 2001, 622, 6-18; J.F. Hartwig, Synlett 1997, 329):

R'NH₂ + R"X → R'NHR"

oder

R"NH₂ + R'X → R'NHR" ,

wobei X für ein Halogenatom, insbesondere für Chlor, Brom oder lod, oder etwa für ein geschütztes Sauerstoffatom, beispielsweise OTf, steht.

Insbesondere kann eines der folgenden Amine, ausgewählt aus der Gruppe bestehend aus N-(4-methylpyrid-2-yl)-N-(pyrimid-2-yl)amin, N-(4,6-dimethylpyrid-2-yl)-N-(6-methoxypyrid-2-yl)amin, N,N-bis(pyrazinyl)amin, N,N,N'-tris(pyrid-2-yl)-o-phenylendiamin als Ausgangsverbindung für die erfindungsgemäßen Liganden verwendet werden. Diese neuen Amine sind ebenfalls Gegenstand der vorliegenden Erfindung.

Die neuen Liganden werden erfindungsgemäß in Kombination mit Übergangsmetallkomplexen oder Übergangsmetallsalzen der Nebengruppe VIII des Periodensystems der Elemente wie beispielsweise Palladium, Nickel, Platin, Rhodium, Iridium, Ruthenium, Cobalt als Katalysatoren eingesetzt.

Dabei können die erfindungsgemäßen Liganden in der Regel in situ zu entsprechenden Übergangsmetallprecusorverbindungen gegeben werden und so für katalytische Anwendungen verwendet werden.

Ein weiterer Gegenstand der vorliegenden Erfindung ist daher eine Komplex-verbindung, umfassend ein erfindungsgemäßes P-funktionalisiertes Amin eines N-haltigen Aromaten sowie ein Metall der Gruppe VIII. Hierbei ist das Übergangsmetall vorzugsweise Teil eines 5-gliedrigen Rings und besonders bevorzugt sind ferner 1 Phosphor-Atom und 2 Stickstoff-Atome Teil dieses 5-gliedrigen Rings.

Aufgrund der Ausbildung eines 5-gliedrigen Ringes, anstelle des normalerweise anzutreffenden 6-gliedrigen Ringes, ist der Komplex besonders gut geeignet auch zur Katalyse von Reaktionen mit Substraten, die sterisch besonders anspruchsvolle Substituenten tragen.

Ein weiterer Gegenstand der vorliegenden Erfindung ist ein Verfahren zur Herstellung der erfindungsgemäßen Komplex-Verbindungen, dadurch gekennzeichnet, daß erfindungsgemäße Liganden, die vorzugsweise wie zuvor beschrieben erhalten werden, mit einem Komplex und/oder Salz eines Übergangsmetalls der Gruppe VIII des Periodensystems umgesetzt werden. Bei dem Übergangsmetall der Gruppe VIII handelt es sich bevorzugt um Pd oder Ni.

Als Palladiumkomponente können mit den erfindungsgemäßen Liganden beispielsweise eingesetzt werden: Palladium(II)acetat, Palladium(II)chlorid, Palladium(II)bromid, Lithiumtetrachloropalladat(II), Palladium(II)acetylacetonat, Palladium(0)-dibenzylidenaceton-Komplexe, insbesondere Dipalladium-trisdibenzylidenaceton, Palladium(1,5-cyclooctadien)chlorid, Palladium(0)tetrakis (triphenylphosphan), Palladium(0)bis(tri-o-tolylphosphan), Palladium(II)propionat, Palladium(II)-bis(triphenylphosphan)dichlorid, Palladium(0)dialtylether-Kompiexe, Palladium(II)nitrat, Palladium(II)chlorid-bis(acetonitril), Palladium(II)chlorid-bis(benzonitril) und weitere Palladium(0)- und Palladium(II)-Komplexe.

Als Nickelvorstufen können beispielsweise Bis(1,5-cyclooctadien)nickel(0), Bis(triphenylphosphin)nickel(II)bromid, Nickel(II)acetat, Nickel(II)chlorid, Nickel(II)acetylacetonat, Nickel(II)bromid, Nickel(0)tetrakis(triphenylphosphan), Nickel(II)iodid, Nickel(II)trifluoracetylacetonat oder Nickelbromid-dimethoxyethanaddukt eingesetzt werden.

Alternativ kann die Herstellung des Komplexes verkürzt werden, indem unmittelbar ausgehend von den Liganden-Vorstufen die Herstellung entweder im Batch-Verfahren oder aber, besonders bevorzugt, in situ erfolgt, ohne daß ein stufenweises Vorgehen und/oder eine Reinigung der erfindungsgemäßen Liganden erforderlich wäre.

Auf diese Weise kann etwa bei Verwendung von Di-tert.-Butylchlorphosphan die Herstellung des Komplexes ausgehend von den Ligandenvorstufen in situ erfolgen, indem gleich zu Beginn sämtliche Reaktionspartner auf einmal vorgelegt werden.

Bei Verwendung von Diphenyl- und Dicyclohexylchlorphosphan erfolgt die Herstellung des Komplexes ausgehend von den Ligandenvorstufen vorzugsweise im Batchverfahren.

Besonders bevorzugt werden auch die entstandenen Komplexe ohne vorherige Reinigung auf Aktivität hin untersucht, indem die Substrate für die auszuführende Reaktion direkt in die bei Herstellung der Komplexe erhaltenen Reaktionslösung hinzugegeben werden.

Auf diese Weise kann also das gesamte Verfahren, ausgehend von den Liganden-Vorstufen bis hin zum Aktivitätstest für die erhaltenen Komplexe im Batch-Verfahren, also in einem Eintopf-Verfahren durchgeführt werden. Dies ist von besonderem Vorteil, da hierdurch effizient in großem Maßstab eine Vielzahl von Komplexe parallel auf ihre katalytische Aktivität hin getestet werden können und desweiteren eine Miniaturisierung des Verfahrens möglich wird. Dies führt letztlich zu einer enormen Zeit- und Kostenersparnis.

Ein besonderer Vorteil der erfindungsgemäßen Liganden und Komplexe ist daher, daß diese effizient und vielfältig mittels Parallelsynthese unter anaeroben Bedingungen dargestellt werden können.

Weiterer Gegenstand der vorliegenden Erfindung ist die Verwendung eines Liganden gemäß Anspruch 1 als Katalysator in Kombination mit Übergangsmetallkomplexen oder Übergangsmetallsalzen der VIII. Nebengruppe des Periodensystems der Elemente, zur Aktivierung von Aryl- oder Vinylhalogeniden für die Herstellung von Dienen oder arylierten Olefinen gemäß der Heck-Reaktion, Biarylen gemäß der Suzuki Reaktion, α-Arylketonen und/oder Aminen aus Arylhalogeniden oder Vinylhalogeniden oder für Carbonylierungen von Arylhalogeniden, Alkinylierungen mit Alkinen gmeäß der Sonogashira-Kupplung und Kreuzkupplungen mit metallorganischen Reagenzien oder für die Herstellung von Aryloefinen, Dienen, Diarylen, Benzosäurederivaten, Acrylsäurederivaten, Arylalkanen, Alkinen und/oder Aminen.

Die so hergestellten Verbindungen können unter anderem eingesetzt werden als UV-Absorber, als Zwischenprodukte für Pharmazeutika und Agrochemikalien, als Ligandvorstufen für Metallocenkatalysatoren, als Riechstoffe, Wirkstoffe und Bausteine für Polymere.

Im allgemeinen wird der Phosphanligand bei katalytischen Anwendungen im Überschuß zum Übergangsmetall eingesetzt. Das Verhältnis Übergangsmetall zu Ligand beträgt vorzugsweise von 1 : 1 bis 1 : 1000. Besonders bevorzugt werden Verhältnisse von Übergangsmetall zu Ligand von 1 : 1 bis 1 : 100. Das genaue zu verwendende Übergangsmetall/Ligand-Verhältnis hängt von der konkreten Anwendung, aber auch von der eingesetzten Katalysatormenge ab.

Die Übergangsmetallkonzentration liegt erfindungsgemäß vorzugsweise zwischen 5 Mol-% und 0,001 Mol-%, besonders bevorzugt zwischen 1 Mol-% und 0,01 Mol-%.

Die erfindungsgemäßen Katalysatoren werden erfindungsgemäß vorzugsweise bei Temperaturen von 0 bis 200 °C eingesetzt.

Ein besonderer Vorteil der erfindungsgemäßen Liganden ist die hohe Aktivität, die die Liganden bei der Aktivierung von kostengünstigen jedoch inerten Chloraromaten induzieren. Wie in den Versuchsbeispielen gezeigt, übertreffen Palladiumkatalysatoren mit den neuen Liganden die bis dato bekannten besten Katalysatorsysteme.

So sind einige der bislang bekannten aktivsten Liganden in der Palladiumkomplex- und/oder Nickelkomplex-katalysierten Suzuki-Kopplung das TtBP (A.F. Littke, G.C. Fu, Angew. Chem. Int. Ed. 1998, 37, 3387-3388; A.F. Littke, C. Dai, G.C. Fu, J. Am. Chem. Soc. 2000, 122, 4020-28) sowie BINAP und BDPP (Wagaw und Buchwal, J. Org. Chem. 1996, 61, 7240-41; J.F. Hartwig, Synlett 1996, 329-340; Silberg et al., J. Organomet. Chem. 2001, 622, 6-18; Schareina et al., Eur. J. Inorg. Chem. 2001, 2421-6). Deren Aktivität wird von den erfindungsgemäßen Katalysatorsystemen reproduzierbar übertroffen, wie auch den Tabellen zu entnehmen.

### Abbildungen:

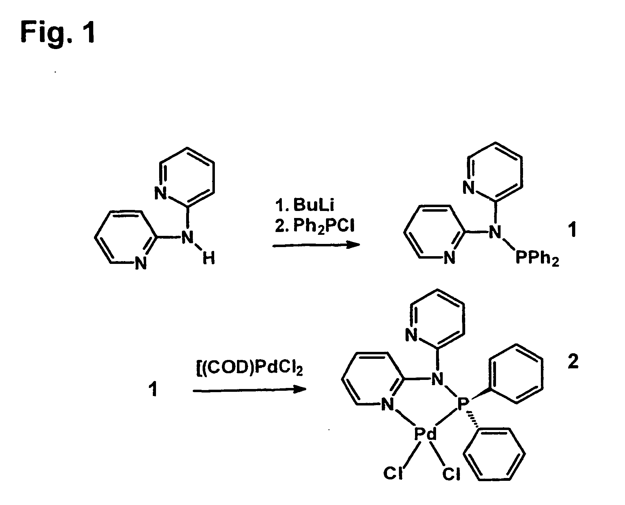

In Fig. 1 ist beispielhaft die Herstellung eines erfindungsgemäßen Liganden und die anschließende Herstellung des entsprechenden Komplexes mit einem Übergangsmetall der Gruppe VIII dargestellt.

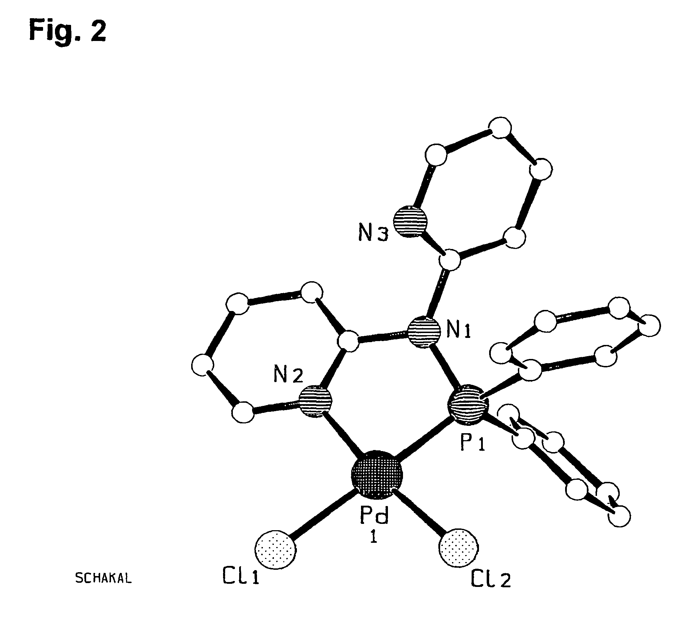

In Fig. 2 ist die Molekülstruktur des gemäß der in Fig. 1 dargestellten Reaktionsschritte erhältlichen Komplexes dargestellt. Die Struktur wurde mittels Röntgenstrukturanalyse ermittelt. Ausgewählte Bindungslängen und Winkel [Å, °]: N1 P1 1.702(5), N2 Pd1 2.053(5), P1 Pd1 2.1951(15), CI1 Pd1 2.3731(14), Cl2 Pd1 2.303(2), N2 Pd1 P1 83.15(14), P1 Pd1 CI2 89.52(6), N2 Pd1 CI1 95.00(14), CI2 Pd1 CI1 92.36(6). Mittelwerten^{[xyz]} für die folgenden Bindungsparameter: Pd-N 2.074(12) Å, Pd-P 2.206(5) Å, Pd-Cl Å 2.33(1) und N-Pd-P 84.7(5) °.

### Ausführungsbeispiele:

### Allgemeines: Materialien und Arbeitsweise:

Die kommerziell erhältlichen Materialien wurden ohne weitere Reinigung verwendet. Luft- und wasserempfindliche Materialien wurden unter striktem Ausschluß von Luft und Wasser in getrockneten Schlenkgefäßen oder in einer Handschuhbox (Firma Braun, Labmaster 130) gehandhabt. Lösungsmittel (Aldrich) und NMR-Lösungsmittel (Cambridge lsotope Laboratories, mind. 99 atom% D) wurden von Natriumtetraethylaluminat bzw. Molsieb (CH₂Cl₂, CD₂Cl₂) destilliert.

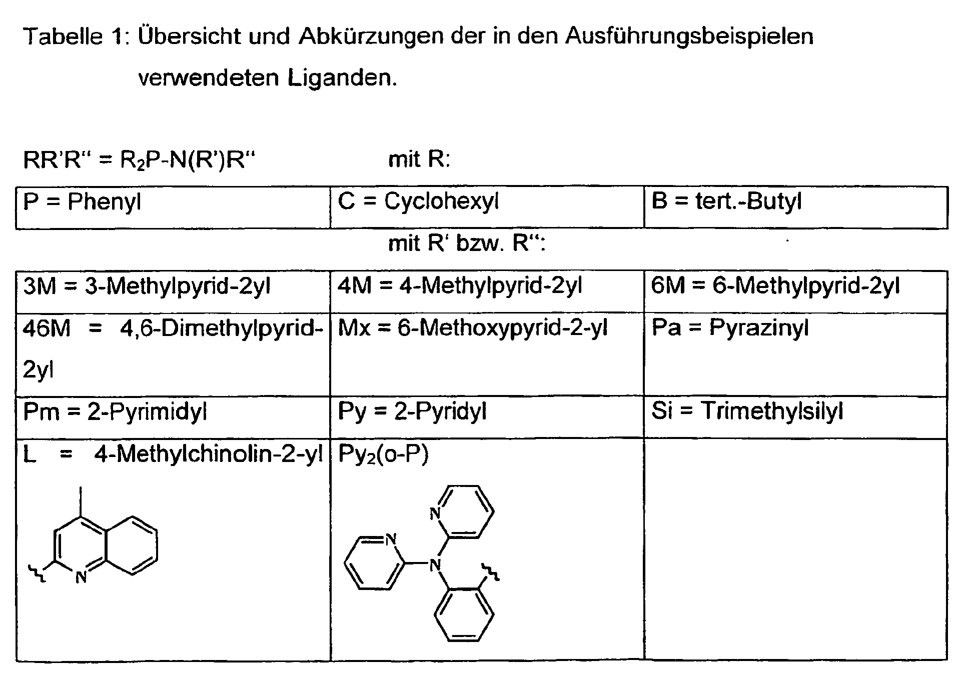

Um einen Vergleich hinsichtlich der Reaktivität zu erhalten, wurden folgende Liganden, die den nächstliegenden Stand der Technik darstellen, eingesetzt: TtBP = Tri(*t*-butyl)phosphan, BINAP = rac.-Bis(diphenylphosphino)-1,1'-binaphtyl, BDPP = 1,3-Bis(diphenylphosphino)propan; DtBPCl = Di-*tert*.-butylphosphinchlorid;

**Tabelle 2: Abkürzungen und Übersicht der erfindungsgemäß verwendeten Metallsalze.**

| | |
|---|---|
| Pd₂(dba)₃ | Dipalladium-tris-dibenzylidenaceton. |
| Pd(OAc)₂ | Palladiumacetat. |
| PdCl₂(cod) | Palladium(1,5-cyclooctadien)chlorid. |
| Ni(cod)₂ | Nickel-bis-(1,5-cyclooctadien). |
| NiBr₂(dme) | Nickelbromid-dimethoxyethan-addukt. |

### Beispiel 1: Bereitstellung und Darstellung von Vorstufen für die Herstellung der erfindungsgemäßen Liganden

Die im folgenden aufgeführten Amine wurden als Vorstufen für die Herstellung der in den Ausführungsbeispielen hergestellten Liganden verwendet.

### 2,2'-Dipyridylamin, Abkürzung -PyPy: kommerziell erhältlich

**Tabelle 3: Bereits bekannte Amine**

| Name | Abkürzung |
|---|---|
| N-(pyrid-2-yl)-N-(3-methylpyrid-2-yl)amin | -3MPy¹ |
| N-(pyrid-2-yl)-N-(6-methylpyrid-2-yl)amin | -6MPy¹ |
| N-(6-methylpyrid-2-yl)-N-(4-methylquinolin-2-yl)amin | -6ML¹ |
| N,N-bis(6-methylpyrid-2-yl)amin | -6M6M¹ |
| N,N-bis(pyrimid-2-yl)amin | -PmPm² |

| | |
|---|---|
| ¹ J. Silberg, T. Schareina, R. Kempe, K. Wurst, M. R. Buchmeiser, *J. Organomet. Chem.*, **2001**, *622*, 6. | |
| ² M. R. Buchmeiser, T. Schareina, R. Kempe, K. Wurst, *J. Organomet. Chem. ,* in Druck. | |

**Tabelle 4: Neue Vorstufen für erfindungsgemäße Liganden:**

| Name | Abkürzung |
|---|---|
| N-(4-methylpyrid-2-yl)-N-(pyrimid-2-yl)amin | -4MPm |
| N-(4,6-dimethylpyrid-2-yl)-N-(6-methoxypyrid-2-yl)amin | -46MMx |
| N, N-bis(pyrazinyl)amin | -PaPa |
| N,N,N'-tris(pyrid-2-yl)-o-phenylendiamin | -Py₂(o-P)Py |

Für die Liganden mit nur einem heterocyclischen Substituenten (z. B. PSiPy, CSiPm etc.) wurden die entsprechenden kommerziell erhältlichen Amine als Startmaterial eingesetzt.

Trimethylsilyl-substituierte Verbindungen wurden aus dem Amin in THF durch Zugabe von 1 Äquivalent n-BuLi-Lösung in Hexan bei -77°C, Aufwärmen auf Raumtemperatur, Zugabe von 1 Äquivalent reinem (CH₃)₃SiCl und 24h Rühren bei Raumtemperatur hergestellt. Weitere Modifikation mit Phosphinen erfolgte analog.

Die erfindungsgemäß eingesetzten Phosphanylreste Diphenylchlorphosphan, Dicyclohexylchlorphosphan und Di-*tert*.-butylchlorphosphan sind alle kommerziell erhältlich.

Die Metallvorstufen waren kommerziell erhältlich oder wurden nach Literaturvorschriften hergestellt.

### a) Herstellung der Vorstufe -4MPm

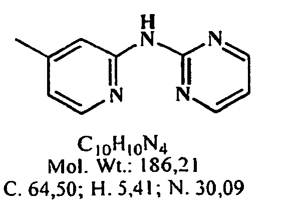

4.33g (40mmol) 2-Amino-4-Picolin
4.58g (40mmol) 2-Chlorpyrimidin
80mg (0.2mmol) Bis-diphenylphosphinopropan
88mg (0.2mmol Pd) Dipalladium-tris-(dibenzylidenaceton)
4.22g (44mmol) Natrium-*tert*.-butylat

Unter Ar in einem 100ml-Schlenk zusammengegeben. Die Mischung wird ohne Lösungsmittel unter Rühren für 24h auf 90°C erwärmt. Schmilzt zu dunkelbrauner Schmelze ohne erkennbare Feststoffbestandteile. Aufarbeitung: Zugabe von Dichlormethan (löst sich vollständig), dann mit Wasser und gesättigter Kochsalzlösung gewaschen, über Natriumsulfat getrocknet und einrotiert. Dunkelrotbraune teerartige Masse. Das Rohprodukt wird mit Dichlormethan auf Kieselgel aufgezogen, in eine Soxhlet-Hülse gefüllt und mit Petroether (Siedebereich 80-100°C)/Toluol, v/v ca. 3:1, für 3d extrahiert. Der nach Abkühlen auftretende Niederschlag wird über eine P4-Fritte filtriert und mit n-Hexan und n-Pentan gewaschen. Auswaage 2.12g, 28% der Th., NMR-sauber.
Elementaranalyse, berechnet für C₁₀H₁₀N₄ (M_{w} = 186.21 g/mol): C 64.50; H 5.41; N 30.09. Gefunden: C 64.47; H 5.30; N 30.31.
¹H-NMR (CDCl₃): 9.16 (bs, 1H, NH), 8.55 (m, 2H), 8.24 (m, 2H), 6.77 (m, 2H), 2.38 (s, 3H, CH₃)
¹³C-NMR (CDCl₃): 159.7, 158.4, 153.2, 150.1, 147.5, 136.1, 119.3, 113.7, 113.5, 22.1.

### b) Herstellung der Vorstufe -46MMx

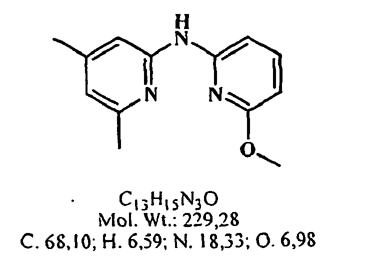

3.00g (24.6mmol) 2-Amino-4,6-dimethylpyridin
2.92ml (3.53g, 24.6mmol) 2-Chlor-6-methoxypyridin
0.082g (0.2mmol) Bis-diphenylphosphinopropan
0.090g (0.2mmol Pd) Dipalladium-tris-(dibenzylidenaceton)
2.88g (30mmol) Natrium-*tert*.-butylat
ca. 20ml Toluol

Die Mischung wird in Ölbad unter Rühren auf 75°C geheizt, nach 2h auf 80°C erhöht. Nach weiteren 30min fällt ein Niederschlag aus. DC-Kontrolle (Lösungsmittel Dichlormethan, Laufmittel PE/Ethylacetat 1:1) zeigt, daß die Reaktion nach 4h noch nicht vollständig ist, daher wird für weitere 12h bei 80°C rühren gelassen. Die Aufarbeitung erfolgt mit Dichlormethan und Wasser, die organische Phase wird über Natriumsulfat getrocknet und zu braunem Öl einrotiert, das beim Stehen über Nacht fest wird. Die gesamte Menge wird aus 20ml n-Hexan umkristallisiert. Nach Auskristallisation werden große Kristallkonglomerate erhalten, die nicht aus dem Kolben zu bekommen waren. Das Lösungsmittel wird abdekantiert, der Feststoff mit n-Hexan gespült, in Dichlormethan gelöst, filtriert, einrotiert. Das Produkt wird im Ölpumpenvakuum geschmolzen, bis keine Lösungsmittel-Gase mehr austreten, und stehengelassen. Rotbraunes Öl, aus dem schnell Kristallnester wachsen. Auswaage 4.81g (85% d. Th.). Analysen o.k.
Elementaranalyse, berechnet für C₁₃H₁₅N₃O (M_{w} = 229.28 g/mol): C 68.10; H 6.59; N 18.33. Gefunden: C 68.24; H 6.64; N 18.08.
¹H (C₆D₆): 7.347 (bs, 1 H, NH); 7.12 (t, 1 H); 6.98 (d, 1 H); 6.92 (s, 1 H); 6.30 (d, 1 H); 6.26 (s, 1 H); 3.77 (s, 3H, -O-CH₃); 2.38 (s, 3H); 1.92 (s, 3H).
¹³C (C₆D₆): 163.8; 156.8; 154.4; 153.3; 148.7; 140.5; 117.1; 109.5; 103.3; 102.2; 53.3; 24.5; 21.2.

### c) Herstellung der Vorstufe -PaPa

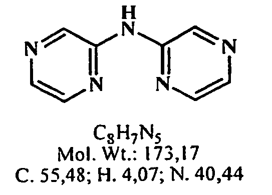

0.951g (10mmol) Aminopyrazin
0.89ml (1.145g, 10mmol) Chlorpyrazin
41mg (0.1mmol) Bis-diphenylphosphinopropan
46mg (0.1mmol Pd) Dipalladium-tris-(dibenzylidenaceton)
1.15g Natrium-*tert*.-butylat

unter Argon in Schlenk einwiegen, dann ca. 20ml abs. Toluol hinzu, bei 80°C in Ölbad rühren. Der Schlenk-Inhalt wird schnell gelb, Niederschlag. Aufarbeitung nach 4h. Die Reaktionsmischung wird auf eine Fritte gegeben, der Schlenk mit Ether gespült. Der Feststoff auf der Fritte wird mit Ether, Wasser, und nochmals mit Ether gewaschen. Umkristallisation aus Wasser, mit heißer Filtration. Nach Exsikkatortrocknung über KOH werden 1.03g feine orangegelbe Nadeln erhalten (60% der Th.).
Elementaranalyse, berechnet für C₈H₇N₅ (M_{w} = 173.17 g/mol): C 55.48; H 4.07; N 40.44. Gefunden: C 55.86; H 4.19; N 40.37.
¹H (DMSO-d₆): 9.52 (bs, 1 H, NH); 8.16 (s, 2H); 7.43 (s, 2H); 7.30 (s, 2H).
¹³C (DMSO-d₆): 150.6; 142.1; 136.9; 135.6.

### d) Herstellung der Vorstufe -Py₂(o-P)Py

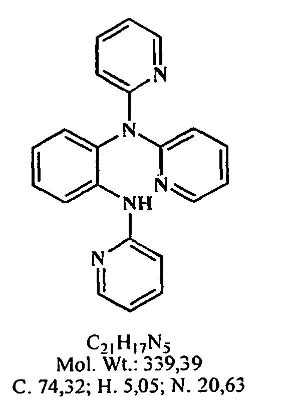

1.08g (10mmol) o-Phenylendiamin
40mg (0.1mmol) Bis-diphenylphosphinopropan
44mg (0.1mmol Pd) Dipalladium-tris-(dibenzylidenaceton)
3.17g (33mmol) Natrium-*tert*.-butylat
2.93ml (4.75g, 30mmol) 2-Brompyridin

20ml Diglyme (Diethylenglykoldimethylether) dazu, auf 110°C bringen, rühren. Nach 2d auf 125°C gestellt, nach insgesamt 6d abgebrochen und aufgearbeitet. Das Lösungsmittel wird in einen Trockeneis-gekühlten Kolben abkondensiert. Wasser und Dichlormethan werden zugegeben, bis beide Phasen klar sind. Die Mischung wird in einen Scheidetrichter umgefüllt. Die organische Phase wird je 1x mit Wasser und gesättigter Kochsalzlösung gewaschen, über Natriumsulfat getrocknet, einrotiert. Die Reinigung erfolgt durch Säulenchromatographie auf Kieselgel 60 der Fa. Merck, Laufmittel ist Ethylacetat. Das Zielprodukt wird nach dem Zwischenprodukt N,N'-bis(pyrid-2-yl)-o-phenylendiamin eluiert. Nach Entfernen der Lösungsmittel beträgt die Auswaage 1.70g (50% der Th.).
Elementaranalyse, berechnet für C₂₁H₁₇N₅ (M_{w} = 339.39 g/mol): C 74.32; H 5.05; N 20.63. Gefunden: C 74.33; H 4.91; N 20.76.
¹H (CDCl₃): 8.22 (dd, 2H); 8.10 (d, 1 H); 8.03 (m, 1 H); 7.46 (m, 2H); 7.38 (bs, 1H). NH); 7.31 (m, 2H); 7.23 (dd, 1H); 7.03 (m, 1H); 6.91 (d, 2H); 6.82 (m, 2H); 6.59 (m, 2H).
¹³C: 157.3; 155.4; 148.3; 137.8; 137.4; 130.3; 128.3; 123.3; 121.6; 118.1; 115.8; 114.8; 110.1.

### Beispiel 2: Schrittweise Synthese von Ligand und Komplex anhand Verbindung 1 und Komplex 2

### a) Herstellung des Liganden Ph₂P-N(Pyridyl)₂

Zu 0.68g (4 mmol) Bipyrid-2-ylamin in 10ml Ether unter Argon werden bei -77°C 1.6ml 2.5M (4 mmol) n-BuLi in Hexan gegeben. Nach 1 h werden 0.72ml (0.882g, 4mmol) Chlordiphenylphosphin in 4ml Ether tropfenweise zugefügt. Der Schlenkinhalt wird beim Zutropfen leuchtend gelb. Nach Rühren über 48h bei Raumtemperatur wird die Lösung durch Filtration in einen anderen Schlenk überführt, dann wird 2x mit einer Mischung aus je 10ml Ether und 10ml THF nachgewaschen. Die Lösungsmittel werden im Vakuum in einen mit Trockeneis gekühlten Kolben abkondensiert, der Feststoff danach bei 1 mbar und Raumtemperatur getrocknet. Auswaage 1.42g (quantitativ) NMR-reine Substanz. Eine Probe wird in Diethylether gelöst und mit n-Hexan überschichtet. Nach einiger Zeit fallen farblose Kristalle aus, die in der Röntgenstrukturanalyse untersucht werden.
Elementaranalyse: C₂₂H₁₈N₃P; Molgewicht: 355.37 g·mcl⁻¹; Ber.: C. 74.35; H. 5.11; N. 11.82; Gef.: C 74.50; H 5.31; N 11.68.
¹H (C₆D₆): 8.16 (m, 2H); 7.73 (m, 4H); 6.97 (m, 6H); 6.57 (d, 2H); 6.33 (ddd, 2H).
¹³C (C₆D₆): 158.35; 158.29; 148.81; 138.74; 138.54; 136.97; 136.07; 136.06; 133.76; 133.55; 132.04; 128.77; 128.53; 128.11;; 118.37; 118.26.
³¹P (C₆D₆): 69.37.

### b) Herstellung des Pd-Komplexes von Ph₂P-N(Pyridyl)₂

Zu 0.071g (0.25mmol) (COD)PdCl₂ und 0.089g Ph₂P-N(Pyridyl)₂ in einem Schlenkgefäß werden unter Argon 4ml trockenes Dichlormethan hinzugegeben. Die Feststoffe lösen sich sehr schnell. Durch Überschichten mit 6ml Diethylether werden nach ca. 1 Woche blaßgelbe Kristalle erhalten. Isolierung durch Filtration, mit 4ml Ether gewaschen, im Ölpumpenvakuum getrocknet. Gelbe Kristalle, 0.10g (75% der Th.).
Elementaranalyse: C₂₂H₁₈Cl₂N₃PPd; Molgewicht: 532,70 g·mol⁻¹; Ber.: C. 49,60; H. 3,41; N. 7,89; Gef.: C 49.29; H 3.51, N 7.81.
¹H (CD₂Cl₂): 9.43 (m, 1 H); 8.24 (m, 1H): 7.88 (m, 5H); 7.66 (m, 1H); 7.50 (m, 4H); 7.37 (m, 5H); 7.08 (m, 1 H); 7.02 (m, 1 H); 6.70(m, 1 H); 6.54 (m, 1 H).
¹³C (CD₂Cl₂): 150.4; 150.1; 149.1; 140.2; 138.5; 133.4; 133.3; 132.2; 127.8; 127.7; 125.9; 125.3; 122.8; 121.7; 121.7; 120.8; 118.0.
³¹P (CD₂Cl₂): 99.2.

### Beispiel 3: Durchführung ausgewählter Suzuki-Reaktionen

Alle Lösungsmittel werden mit Standardmethoden getrocknet, von Na-Benzophenonketyl und Natriumtetraethylat destilliert und unter Argon aufbewahrt. Kommerziell erhältliche Startmaterialien wurden ohne weitere Reinigung eingesetzt, Flüssigkeiten unter Argon aufbewahrt. Alle Operationen fanden in Schlenkgefäßen unter Argon statt.

Stammlösungen der Substrate, Liganden und Metallvorstufen werden in dem angegebenen Lösungsmittel hergestellt, die Konzentration betrug für Substrate (Chloraromaten und Phenylboronsäure) 1 mol l⁻¹, für Liganden und Metallvorstufen, c = 0.025 mol l⁻¹.

Die Liganden wurden im Fall der diphenyl- und dicyclohexylsubstituierten Phosphanylreste wie folgt hergestellt: das Amin (1mmol) wird mit 0.4ml einer 2.5M Lösung n-BuLi in Hexan bei -77°C metalliert, für 30min bei -77°C gehalten, dann auf Raumtemperatur aufwärmen gelassen, dann wird ein Äquivalent Chlordiphenylphosphan bzw. Chlordicyclohexylphosphan zugefügt und für mind. 24h bei Raumtemperatur gerührt. Die Lösung wird mit inertem Lösungsmittel aufgefüllt, so daß die Konzentration des Liganden 0,025 mol l⁻¹ beträgt.

Die Herstellung der di(*tert*.-butyl)phosphansubstituierten Liganden erfolgte in situ, indem im Reaktionsschlenk je 1 Äquivalent Amin, Chlor-di(*tert*.-butyl)phosphan und Metallvorstufe jeweils als Stammlösungen zusammen mit der Base für 1h bei 60°C erhitzt werden, danach erfolgt bei Raumtemperatur die Zugabe der Substrate.

Die Screeningreaktionen wurden wie folgt durchgeführt: Alle Reaktionen wurden in der Parallelapparatur unter Ar durchgeführt. Die Base wurde zuvor im Vakuum bei 130°C für 24h getrocknet, die Einwaage erfolgte in einer Vakuumbox. Dann wurden Stammlösungen der Substrate (Halogenaromaten 1 ml, Phenylboronsäure 1,2 ml) und Reagenzien (Liganden und Metallvorstufen, c = 0,025 mol l⁻¹) zugefügt und unter Rühren auf die Reaktionstemperatur gebracht.
Nach Ablauf der Reaktionszeit wurden aliquote Proben entnommen, ein interner Standard zugefügt (Dodekan), mit Diethylether verdünnt und mittels Gaschromatographie analysiert. Als Nebenprodukt war in jedem Fall Biphenyl angegeben.
Bibliothek 1: Lösungsmittel THF; 1,2 mmol Base; 1 mmol 4-Chlorbenzonitril; 1,2 mmol Phenylboronsäure; 1 % Metall(vorstufe); 1 Äquivalent (relativ zum Metall) Ligand.
Bibliothek 2: Lösungsmittel THF; 1,2 mmol Base, 1 mmol 4-Chforanisol; 1,2 mmol Phenylboronsäure; 1 % Metall(vorstufe); 1 Äquivalent (relativ zum Metall) Ligand; 60°C; 24 Stunden.
Bibliothek 3: Lösungsmittel 1,4-Dioxan; 1,2 mmol Base; 1 mmol 3-Chlorpyridin; 1,2 mmol Phenylboronsäure; 1 % Metall(vorstufe); 1 Äquivalent (relativ zum Metall) Ligand; 60°C; 24 Stunden

### Tabelle 5: Übersicht über die Ergebnisse zu folgender Reaktion:

Bibliothek 1: 4-Chlorbenzonitril + Phenylboronsäure → 4-Cyanobiphenyl.

Die Ergebnisse sind nach Ausbeute geordnet.

| Nr | Base | Ligand | Metall | Ausbeute Produkt |
|---|---|---|---|---|
| 01 | K₂CO₃ | B4MPm | Pd₂(dba)₃ | 100 |
| 02 | NaOtBu | BPyPy | Pd₂(dba)₃ | 100 |
| 03 | NaOtBu | B4MPm | Pd₂(dba)₃ | 100 |
| 04 | K₂CO₃ | BPyPy | Pd₂(dba)₃ | 83 |
| 05 | K₂CO₃ | TtBP | Pd₂(dba)₃ | 80 |
| 06 | K₃PO₄ | BPy₂(o-P)Py | Pd(OAc)₂ | 78 |
| 07 | NaOtBu | DtBPCl | Pd₂(dba)₃ | 76 |
| 08 | K₃PO₄ | PPmPm | Ni(cod)₂ | 65 |
| 09 | K₂CO₃ | C4MSi | Pd₂(dba)₃ | 60 |
| 10 | K₃PO₄ | TtBP | Pd₂(dba)₃ | 59 |
| 11 | K₂CO₃ | CPmSi | Pd₂(dba)₃ | 58 |
| 12 | K₃PO₄ | CPy₂(o-P)Py | Ni(cod)₂ | 52 |
| 13 | K₂CO₃ | DtBPCI | Pd₂(dba)₃ | 48 |
| 14 | K₃PO₄ | CPyPy | Pd₂(dba)₃ | 46 |
| 15 | Na₂CO₃ | TtBP | Pd₂(dba)₃ | 43 |
| 16 | K₃PO₄ | CPmPm | Ni(cod)₂ | 42 |
| 17 | K₃PO₄ | CPyPy | NiBr₂(dme) | 35 |
| 18 | K₃PO₄ | CPy₂(o-P)Py | Pd(OAc)₂ | 32 |
| 19 | Na₂CO₃ | CPyPy | Pd₂(dba)₃ | 29 |
| 20 | NaOtBu | BPmSi | Pd₂(dba)₃ | 28 |
| 21 | K₂CO₃ | CPaSi | Pd₂(dba)₃ | 27 |
| 22 | K₃PO₄ | BPmPm | Pd(OAc)₂ | 23 |
| 23 | K₃PO₄ | TtBP | Ni(cod)₂ | 21 |
| 24 | Na₂CO₃ | CPyPy | NiBr₂(dme) | 21 |
| 25 | K₂CO₃ | BPmSi | Pd₂(dba)₃ | 21 |
| 26 | K₂CO₃ | PPmSi | Ni(cod)₂ | 19 |
| 27 | K₃PO₄ | PPy₂(o-P)Py | Ni(cod)₂ | 19 |
| 28 | NaOtBu | BPaSi | Pd₂(dba)₃ | 19 |
| 29 | NaOtBu | B4MPm (0.5%) | Pd₂(dba)₃ | 16 |
| 30 | K₂CO₃ | BPaSi | Pd₂(dba)₃ | 15 |
| 31 | NaOtBu | CPmSi | Pd₂(dba)₃ | 13 |
| 32 | K₃PO₄ | BPy₂(o-P)Py | Ni(cod)₂ | 13 |
| 33 | Na₂CO₃ | TtBP | NiBr₂(dme) | 11 |
| 34 | K₃PO₄ | -- | Ni(cod)₂ | 10 |
| 35 | NaOtBu | B4MPm (0.5%) | Pd₂(dba)₃ | 10 |
| 36 | K₂CO₃ | PaSiP | Ni(cod)₂ | 10 |
| 37 | NaOtBu | BPyPy (0.5%) | Pd₂(dba)₃ | 9 |
| 38 | K₃PO₄ | CPmPm | Pd(OAc)₂ | 8 |
| 39 | K₂CO₃ | B4MPm (0.5%) | Pd₂(dba)₃ | 6 |
| 40 | K₂CO₃ | BPyPy (0.5%) | Pd₂(dba)₃ | 4 |
| 41 | K₃PO₄ | PPy₂(o-P)Py | Pd(OAc)₂ | 4 |
| 42 | NaOtBu | CPmSi (0.5%) | Pd₂(dba)₃ | 3 |
| 43 | K₂CO₃ | C4MSi | NiBr₂(dme) | 3 |
| 44 | K₂CO₃ | PPmSi | Pd₂(dba)₃ | 1 |
| 45 | K₃PO₄ | PPmPm | Pd(OAc)₂ | 1 |
| 46 | K₃PO₄ | BPmPm | Ni(cod)₂ | 1 |
| 47 | K₂CO₃ | PPaSi | Pd₂(dba)₃ | 1 |
| 48 | K₃PO₄ | TtBP | NiBr₂(dme) | 0 |
| 49 | Na₂CO₃ | CPyPy | Ni(cod)₂ | 0 |
| 50 | Na₂CO₃ | TtBP | Ni(cod)₂ | 0 |
| 51 | K₂CO₃ | TtBP | NiBr₂(dme) | 0 |
| 52 | K₂CO₃ | C4MSi | Ni(cod)₂ | 0 |
| 53 | K₂CO₃ | TtBP | Ni(cod)₂ | 0 |
| 54 | NaOtBu | PPmSi | Pd₂(dba)₃ | 0 |
| 55 | NaOtBu | PPaSi | Pd₂(dba)₃ | 0 |
| 56 | K₂CO₃ | BPmSi | Ni(cod)₂ | 0 |
| 57 | K₂CO₃ | CPmSi | Ni(cod)₂ | 0 |
| 58 | K₂CO₃ | BPaSi | Ni(cod)₂ | 0 |
| 59 | K₂CO₃ | CPaSi | Ni(cod)₂ | 0 |
| 60 | NaOtBu | CPaSi | Pd₂(dba)₃ | 0 |

### Tabelle 6: Übersicht über die Ergebnisse zu folgender Reaktion:

Bibliothek 2: 4-Chloranisol + Phenylboronsäure → 4-Methoxybiphenyl.

Die Ergebnisse sind nach Ausbeute geordnet.

| Nr. | Base | Ligand | Metall | Ausbeute Produkt | Ausbeute Nebenprodukt |
|---|---|---|---|---|---|
| 01 | K₃PO₄ | C46MMx | Ni(cod)₂ | 53 | 14 |
| 02 | K₂CO₃ | BPmPm (1 eq) | Pd₂(dba)₃ | 53 | 6 |
| 03 | Na₂CO₃ | PPh₃ | Ni(cod)₂ | 43 | 3 |
| 04 | K₃PO₄ | PPh₃ | Ni(cod)₂ | 38 | 12 |
| 05 | K₃PO₄ | BPmPm | Pd₂(dba)₃ | 37 | 0 |
| 06 | K₃PO₄ | C6MPy | Ni(cod)₂ | 35 | 14 |
| 07 | K₂CO₃ | B4MPm | Pd₂(dba)₃ | 35 | 0 |
| 08 | K₃PO₄ | C6M6M | Ni(cod)₂ | 32 | 6 |
| 09 | K₂CO₃ | C46MMx | Ni(cod)₂ | 31 | |
| 10 | K₃PO₄ | BPmSi | Ni(cod)₂ | 31 | 0 |
| 11 | Na₂CO₃ | C46MMx | Ni(cod)₂ | 29 | |
| 12 | K₂CO₃ | BPmSi | Pd₂(dba)₃ | 26 | 4 |
| 13 | K₃PO₄ | B4MPm | Pd₂(dba)₃ | 26 | |
| 14 | K₃PO₄ | P6M6M | Ni(cod)₂ | 24 | 16 |
| 15 | K₃PO₄ | P6ML | Ni(cod)₂ | 24 | 15 |
| 16 | K₃PO₄ | 46MMxP | Ni(cod)₂ | 24 | 15 |
| 17 | K₃PO₄ | BPmSi | Pd₂(dba)₃ | 19 | 3 |
| 18 | K₃PO₄ | P46ML | Ni(cod)₂ | 17 | 23 |
| 19 | K₃PO₄ | PPyH | Ni(cod)₂ | 17 | 12 |
| 20 | K₃PO₄ | C46MMx | Pd₂(dba)₃ | 14 | 3 |
| 21 | Na₂CO₃ | C46MMx | Pd₂(dba)₃ | 12 | |
| 22 | K₃PO₄ | P6MPy | Ni(cod)₂ | 11 | 16 |
| 23 | K₃PO₄ | C6M6M | Pd₂(dba)₃ | 11 | 3 |
| 24 | K₂CO₃ | C46MMx | Pd₂(dba)₃ | 11 | |
| 25 | K₃PO₄ | DtBPCl | Pd₂(dba)₃ | 11 | 5 |
| 26 | K₂CO₃ | DtBPCI | Pd₂(dba)₃ | 9 | 0 |
| 27 | K₃PO₄ | C6MPy | Pd₂(dba)₃ | 8 | 3 |
| 28 | K₂CO₃ | -4MPm | Pd₂(dba)₃ | 7 | 0 |
| 29 | K₃PO₄ | C3MPy | Pd₂(dba)₃ | 6 | 2 |
| 30 | K₂CO₃ | -PyPy | Pd₂(dba)₃ | 6 | 0 |
| 31 | K₃PO₄ | TtBP | Ni(cod)₂ | 6 | |
| 32 | K₃PO₄ | CPyPy | Pd₂(dba)₃ | 5 | 2 |
| 33 | K₃PO₄ | CPyPy | Pd(OAc)₂ | 5 | 0 |
| 34 | K₃PO₄ | P3MPy | Ni(cod)₂ | 4 | 28 |
| 35 | K₃PO₄ | CPyPy | Ni(cod)₂ | 4 | 10 |
| 36 | K₃PO₄ | C3MPy | Ni(cod)₂ | 4 | 16 |
| 37 | K₃PO₄ | TtBP | Pd₂(dba)₃ | 4 | |
| 38 | K₃PO₄ | DtBPCI | Ni(cod)₂ | 4 | 10 |
| 39 | K₃PO₄ | B4MPm | Ni(cod)₂ | 4 | 32 |
| 40 | Na₂CO₃ | CPyPy | Pd(OAc)₂ | 3 | 0 |
| 41 | K₃PO₄ | PPyPy | Ni(cod)₂ | 2 | 22 |
| 42 | K₃PO₄ | P6MPy | Pd₂(dba)₃ | 2 | 5 |
| 43 | K₃PO₄ | P46ML | Pd₂(dba)₃ | 2 | 6 |
| 44 | K₃PO₄ | PPyPy | Pd₂(dba)₃ | 1 | 4 |
| 45 | K₃PO₄ | P3MPy | Pd₂(dba)₃ | 1 | 4 |
| 46 | K₃PO₄ | P6M6M | Pd₂(dba)₃ | 1 | 6 |
| 47 | K₃PO₄ | P6ML | Pd₂(dba)₃ | 1 | 6 |
| 48 | K₃PO₄ | P46MMx | Pd₂(dba)₃ | 1 | 7 |
| 49 | K₃PO₄ | PPh₃ | Pd₂(dba)₃ | 1 | 6 |
| 50 | Na₂CO₃ | CPyPy | Pd₂(dba)₃ | 1 | 1 |
| 51 | K₃PO₄ | PPyH | Pd₂(dba)₃ | 0 | 6 |
| 52 | K₃PO₄ | CPyH | Pd₂(dba)₃ | 0 | 2 |
| 53 | K₃PO₄ | CPyH | Ni(cod)₂ | 0 | 0 |
| 54 | Na₂CO₃ | CPyPy | Ni(cod)₂ | 0 | 8 |
| 55 | Na₂CO₃ | CPyPy | CoCl₂ | 0 | 0 |
| 56 | Na₂CO₃ | CPyPy | FeCl₂ | 0 | 0 |
| 57 | Na₂CO₃ | CPyPy | CuCl(cod) | 0 | 0 |
| 58 | Na₂CO₃ | PPh₃ | Pd₂(dba)₃ | 0 | 23 |
| 59 | K₃PO₄ | CPyPy | CoCl₂ | 0 | 0 |
| 60 | K₃PO₄ | CPyPy | FeCl₂ | 0 | 0 |
| 61 | K₃PO₄ | CPyPy | CuCl(cod) | 0 | 0 |
| 62 | K₂CO₃ | CPmSi | Pd₂(dba)₃ | 0 | 0 |
| 63 | K₂CO₃ | -PyPy | Ni(cod)₂ | 0 | 0 |
| 64 | K₂CO₃ | B4MPm | Ni(cod)₂ | 0 | 0 |
| 65 | K₂CO₃ | DtBPCI | Ni(cod)₂ | 0 | 0 |
| 66 | K₂CO₃ | BPmPm (1eq) | Ni(cod)₂ | 0 | 0 |
| 67 | K₂CO₃ | BPmSi | Ni(cod)₂ | 0 | 0 |
| 68 | K₂CO₃ | CPmSi | Ni(cod)₂ | 0 | 0 |
| 69 | K₂CO₃ | -PyPy | Pd₂(dba)₃ | 0 | 0 |
| 70 | K₂CO₃ | -PmPm (1eq) | Pd₂(dba)₃ | 0 | 0 |
| 71 | K₂CO₃ | -PmSi | Pd₂(dba)₃ | 0 | 0 |
| 72 | K₂CO₃ | CPmSi | Pd₂(dba)₃ | 0 | 0 |
| 73 | K₂CO₃ | TtBP | Pd₂(dba)₃ | 0 | 13 |
| 74 | K₂CO₃ | -PyPy | Ni(cod)₂ | 0 | 0 |
| 75 | K₂CO₃ | -4MPm | Ni(cod)₂ | 0 | 0 |
| 76 | K₂CO₃ | BPmPm (1eq) | Ni(cod)₂ | 0 | 0 |
| 77 | K₂CO₃ | -PmSi | Ni(cod)₂ | 0 | 0 |
| 78 | K₂CO₃ | CPmSi | Ni(cod)₂ | 0 | 0 |
| 79 | K₂CO₃ | TtBP | Ni(cod)₂ | 0 | 0 |
| 80 | K₃PO₄ | -PmSi | Pd₂(dba)₃ | 0 | |
| 81 | K₃PO₄ | -4MPm | Pd₂(dba)₃ | 0 | |
| 82 | K₃PO₄ | BPmPm | Ni(cod)₂ | 0 | 0 |
| 83 | K₃PO₄ | -PmSi | Ni(cod)₂ | 0 | 0 |
| 84 | K₃PO₄ | -4MPm | Ni(cod)₂ | 0 | 0 |

### Tabelle 7: Übersicht über die Ergebnisse zu folgender Reaktion:

Bibliothek 3: 3-Chlorpyridin + Phenylboronsäure → 3-Phenylpyridin.

Die Ergebnisse sind nach Ausbeute geordnet.

| Nr | Base | Ligand | Metall | Ausbeute Produkt | Ausbeute Nebenprodukt |
|---|---|---|---|---|---|
| 1 | K₃PO₄ | B4MPm | Pd(OAc)₂ | 90 | 43 |
| 2 | K₃PO₄ | B4MPm | Pd₂(dba)₃ | 89 | 36 |
| 3 | K₃PO₄ | B4MPm (1%) | Pd₂(dba)₃ (1%) | 88 | 31 |
| 4 | K₃PO₄ | BPyPy | Pd₂(dba)₃ | 86 | 58 |
| 5 | K₃PO₄ | BPaPa | Pd₂(dba)₃ | 79 | 34 |
| 6 | K₃PO₄ | B4MPm (0.5%) | Pd₂(dba)₃ (0.5%) | 76 | 21 |
| 7 | K₂CO₃ | B4MPm | Pd(OAc)₂ | 74 | 24 |
| 8 | K₃PO₄ | B4MPm (0.25%) | Pd₂(dba)₃ (0.25%) | 69 | 10 |
| 9 | K₂CO₃ | BPyPy | Pd₂(dba)₃ | 58 | 37 |
| 10 | K₂CO₃ | BPyPy | PdCl₂(cod) | 52 | 10 |
| 11 | K₂CO₃ | B4MPm | PdCl₂(cod) | 51 | 11 |
| 12 | K₃PO₄ | BPyPy | PdCl₂(cod) | 42 | 15 |
| 13 | K₂CO₃ | B4MPm | Pd₂(dba)₃ | 39 | 8 |
| 14 | K₃PO₄ | B4MPm | PdCl₂(cod) | 37 | 8 |
| 15 | K₃PO₄ | BPmSi | Ni(cod)₂ | 35 | 16 |
| 16 | K₃PO₄ | BPmSi | Pd₂(dba)₃ | 32 | 19 |
| 17 | K₃PO₄ | BPaPa | PdCl₂(cod) | 31 | 10 |
| 18 | K₂CO₃ | B4MPm | Ni(cod)₂ | 27 | 35 |
| 19 | K₃PO₄ | CPyPy | Ni(cod)₂ | 25 | 33 |
| 20 | K₂CO₃ | DtBPCl | Pd₂(dba)₃ | 20 | 18 |
| 21 | K₃PO₄ | PPyPy | Ni(cod)₂ | 18 | 14 |
| 22 | K₂CO₃ | B4MPm (0.5%) | Ni(cod)₂ (0.5%) | 16 | 31 |
| 23 | K₂CO₃ | PPyPy | Ni(cod)₂ | 10 | 18 |
| 24 | K₂CO₃ | CPyPy | Ni(cod)₂ | 9 | 17 |
| 25 | K₃PO₄ | B4MPm | Ni(cod)₂ | 8 | 11 |
| 26 | K₃PO₄ | C4MPm | Ni(cod)₂ | 7 | 7 |
| 27 | K₃PO₄ | BPyPy | Ni(cod)₂ | 6 | 16 |
| 28 | K₃PO₄ | TtBP | Pd₂(dba)₃ | 5 | 34 |
| 29 | K₂CO₃ | TtBP | Pd₂(dba)₃ | 5 | 38 |
| 30 | K₃PO₄ | BPaPa | Ni(cod)₂ | 5 | 16 |
| 31 | K₃PO₄ | BPaPa | Pd(OAc)₂ | 4 | 1 |
| 32 | K₃PO₄ | C4MPm | Pd₂(dba)₃ | 3 | 9 |
| 33 | K₃PO₄ | rac. BINAP | Pd₂(dba)₃ | 1 | 6 |
| 34 | K₂CO₃ | C4MPm | Pd₂(dba)₃ | 1 | 1 |
| 35 | K₃PO₄ | BDPP | Pd₂(dba)₃ | 0 | 4 |
| 36 | K₂CO₃ | rac. BINAP | Pd₂(dba)₃ | 0 | 7 |
| 37 | K₂CO₃ | BDPP | Pd₂(dba)₃ | 0 | 13 |
| 38 | K₃PO₄ | PPyPy | Pd₂(dba)₃ | 0 | 2 |
| 39 | K₃PO₄ | CPyPy | Pd₂(dba)₃ | 0 | 1 |
| 40 | K₂CO₃ | PPyPy | Pd₂(dba)₃ | 0 | 5 |
| 41 | K₂CO₃ | CPyPy | Pd₂(dba)₃ | 0 | 2 |
| 42 | K₂CO₃ | -4MPm | Pd₂(dba)₃ | 0 | 3 |
| 43 | K₂CO₃ | BPyPy | Ni(cod)₂ | 0 | 4 |
| 44 | K₂CO₃ | C4MPm | Ni(cod)₂ | 0 | 2 |
| 45 | K₃PO₄ | C4MPm | Pd(OAc)₂ | 0 | 4 |
| 46 | K₂CO₃ | C4MPm | Pd(OAc)₂ | 0 | 1 |
| 47 | K₃PO₄ | BPyPy | Pd(OAc)₂ | 0 | 2 |
| 48 | K₂CO₃ | BPyPy | Pd(OAc)₂ | 0 | 2 |

### Beispiel 4: Grignardkupplungen:

Die Durchführung der Versuche erfolgt wie in den beschriebenen Suzuki-Reaktionen in dem Parallelreaktor. Die Reaktionstemperatur betrug 60°C, wenn nicht anders angegeben, die Reaktionszeit 24h. Lösungsmittel ist in allen Fällen THF. Die molaren Verhältnisse der Reaktionspartner betrugen:
Substrat: 1 mmol (1 M Lösung).
Reagenz (Phenylmagnesiumbromid): 1.2mmol (1M Lösung).
Liganden: 0.01mmol (0.02mmol bei monodentaten Liganden), wenn nicht anders angegeben, 0.4ml (0.025M Lösung).
Metallvorstufen: 0.01mmol, (0.025M Lösung).

Nach Ablauf der 24h wurden die Schlenks abkühlen gelassen und je 0.5ml Methanol zur Vernichtung überschüssigen Reagenzes zugefügt. Danach erfolgte die Bestimmung der Ausbeuten mit GC.

### a) Kreuzkupplung: 2-Chlor-m-xylol + Phenylmagnesiumbromid

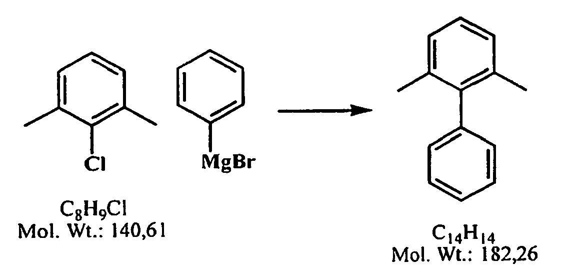

**Tabelle 8: Ergebnisse zu a)**

| Nr | Metall | Ligand | T/°C | Ausbeute Produkt |
|---|---|---|---|---|
| 01 | Ni(0) | B4MPm | 60 | 68 |
| 02 | Ni(0) | B4MPm | 60 | 63 |
| 03 | Ni(0) | BPmPm | 60 | 51 |
| 04 | Ni(0) | B4M4M | 60 | 48 |
| 05 | Ni(0) | DtBPCI | 60 | 29 |
| 06 | Ni(0) | TtBP (4eq) | 60 | 17 |
| 07 | Ni(0) | TtBP (3eq) | 60 | 12 |
| 08 | Ni(0) | TtBP (2eq) | 60 | 10 |
| 09 | Ni(0) | PCy3 | 60 | 10 |
| 10 | Ni(0) | B4MPm | RT | 7 |
| 11 | Pd(0) | TtBP (3eq) | 60 | 4 |
| 12 | Ni(0) | C12PmPm | 60 | 4 |
| 13 | Ni(0) | -- | 60 | 4 |
| 14 | Pd(0) | TtBP (2eq) | 60 | 3 |
| 15 | Pd(0) | TtBP (4eq) | 60 | 3 |
| 16 | Ni(0) | BPmPm | RT | 3 |
| 17 | NiCl2 5% | -- | 60 | 3 |
| 18 | Ni(CO)2(PPh3)2 | -- | 60 | 2 |
| 19 | Ni(0) | PPh3 | 60 | 1 |
| 20 | Ni(0) | DtBPCl | RT | 1 |
| 21 | Pd(0) | DtBPCl | 60 | 0 |
| 22 | CoCl2 | -- | 60 | 0 |
| 23 | Ni(0) | TtBP | RT | 0 |
| 24 | -- | -- | RT | 0 |
| 25 | -- | -- | 60 | 0 |

### b) Kreuzkupplung: 2-Chlortoluol + Phenylmagnesiumbromid

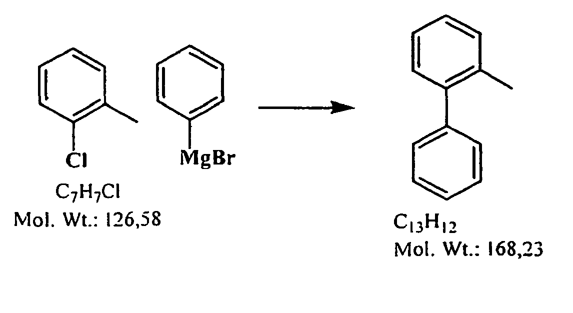

**Tabelle 9: Ergebnisse zu b)**

| Nr | Metall | Ligand | Ausbeute Produkt |
|---|---|---|---|
| 01 | Ni(0) | B4MPm | 88 |
| 02 | Ni(0) | C12-4MPm | 75 |
| 03 | Ni(0) | PCy3 | 65 |
| 04 | Ni(0) | DtBPCI | 58 |
| 05 | Ni(0) | TtBP | 55 |
| 06 | Ni(0) | PPh3 | 53 |
| 07 | Ni(0) | -- | 51 |
| 08 | Ni(0) | C12-4M4M | 51 |
| 09 | Ni(0) | C12-PmPm | 45 |
| 10 | Ni(0) | C12-PmPm | 43 |
| 11 | Ni(0) | BPmPm | 39 |
| 12 | NiCl2 5% | -- | 31 |
| 13 | Pd(0) | TtBP (3eq) | 7 |
| 14 | Pd(0) | TtBP (2eq) | 6 |
| 15 | Pd(0) | TtBP (4eq) | 4 |
| 16 | CoCl2 | C12-4M4M | 3 |
| 17 | CoCl2 | C12-4MPm | 2 |
| 18 | Pd(0) | B4MPm | 2 |
| 19 | CoCl2 | -- | 2 |
| 20 | CoCl2 | C12-PmPm | 1 |
| 21 | Pd(0) | DtBPCl | 0 |

### c) Kreuzkupplung: 2-CIPy + PhMgBr

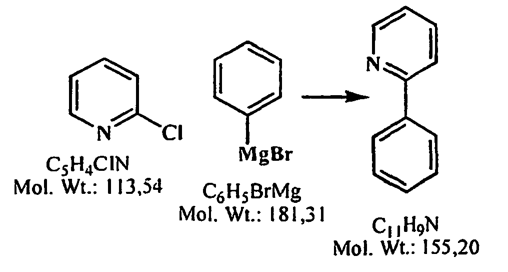

**Tabelle 10: Ergebnisse zu c)**

| Nr. | Metall | Ligand | Ausbeute Prod. |
|---|---|---|---|
| 01 | Pd(0) | B4MPm | 100 |
| 02 | Pd(0) | BPmPm | 97 |
| 03 | Pd(0) | PCy3 | 77 |
| 04 | Ni(0) | TtBP | 73 |
| 05 | Pd(0) | TtBP | 69 |
| 06 | - - | - - | 31 |

### d) Kreuzkupplung: 4-ClAn + Phenylmagnesiumbromid

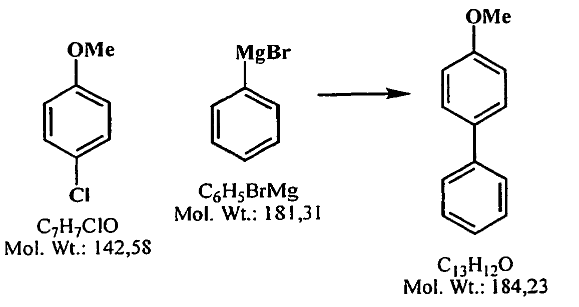

| Nr. | Metall | Ligand | Ausbeute Prod |
|---|---|---|---|
| 01 | Ni(0) | PCy3 | 100 |
| 02 | Ni(0) | TtBP | 100 |
| 03 | Ni(0) | BPmSi | 100 |
| 04 | Ni(0) | CPmSi | 100 |
| 05 | Ni(0) | BPyPy | 100 |
| 06 | Ni(0) | CPyPy | 100 |
| 07 | Ni(0) | -- | 100 |
| 08 | Ni(0) | CAPe | 100 |
| 09 | Ni(0) | B4MPm | 100 |
| 10 | Ni(0) | C4M4M | 99 |
| 11 | Ni(0) | BPmPm | 99 |
| 12 | Ni(0) | CPicSi | 98 |
| 13 | Ni(0) | BPaPa | 97 |
| 14 | Ni(0) | PPmSi | 97 |
| 15 | Ni(0) | PPicSi | 96 |
| 16 | Ni(0) | P4M4M | 96 |
| 17 | Ni(0) | B4M4M | 94 |
| 18 | Ni(0) | -4M4M | 89 |
| 19 | Ni(0) | PPyPy | 84 |
| 20 | Ni(2) | B4MPm | 82 |
| 21 | Ni(0) | BPicSi | 78 |
| 22 | Ni(CO)2(PPh3)2 | -- | 61 |
| 23 | Pd(0) | B4M4M | 51 |
| 24 | Ni(2) | -- | 50 |
| 25 | Ni(2) | PCy3 | 33 |
| 26 | Li2CuCl4 5% | -- | 18 |
| 27 | Pd(0) | BPmPm | 14 |
| 28 | PdAc | PPicSi | 12 |
| 29 | Pd(0) | -4M4M | 10 |
| 30 | Pd(0) | C4M4M | 9 |
| 31 | Pd(0) | TtBP | 8 |
| 32 | PdAc | BPicSi | 7 |
| 33 | Pd(0) | B4MPm | 7 |
| 34 | PdAc | -- | 6 |
| 35 | NiCl2 5% | -- | 2 |
| 36 | -- | -- | 0 |

### e) Kreuzkupplung: 3-CIPy + PhMgBr

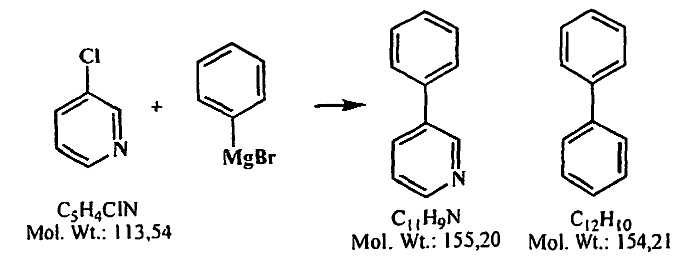

**Tabelle 11: Ergebnisse zu e)**

| Nr. | Metall | Ligand | Ausbeute Produkt |
|---|---|---|---|
| 01 | Ni(0) | BPmPm | 100 |
| 02 | Ni(0) | B4MPm | 96 |
| 03 | Ni(0) | B4M4M | 78 |
| 04 | Ni(0) | P4M4M | 72 |
| 05 | Pd(0) | TtBP | 72 |
| 06 | Ni(0) | C12PmPm 2eq | 68 |
| 07 | NiC12 | DCMA (6eq) | 67 |
| 08 | Pd(0) | C12PmPm 2eq | 61 |
| 09 | Ni(0) | C4M4M | 60 |
| 10 | Ni(0) | CAPe | 59 |
| 11 | Pd(0) | CAPe | 55 |
| 12 | Pd(0) | P4M4M | 50 |
| 13 | PdAc | DCMA (6eq) | 49 |
| 14 | Ni(0) | DtBPCI | 48 |
| 15 | Pd(0) | B4M4M | 45 |
| 16 | Ni(0) | TtBP | 43 |
| 17 | Ni(0) | -4M4M | 43 |
| 18 | Ni(0) | PCy3 | 41 |
| 19 | Pd(0) | C4M4M | 40 |
| 20 | Li2CuCl4 5% | - - | 35 |
| 21 | Pd(0) | PCy3 | 25 |
| 22 | - - | - - | 21 |

## Patentansprüche

1. P-funktionalisierte Amine N-haltiger Aromaten der allgemeinen Formel
R¹(R²)P-N(R')R" (I)
worin
R¹ und R² unabhängig voneinander für einen Rest ausgewählt aus der Gruppe bestehend aus C₁-C₂₄ Alkyl, C₃-C₈ Cycloalkyl und aromatische Reste mit 5 bis 14 C-Atomen stehen, und wobei die Alkyl- und Cycloalkylreste, verzweigt oder unverzweigt sein können,
wobei
die vorgenannten Reste selbst jeweils ein- oder mehrfach substituiert sein können durch Reste unabhängig voneinander ausgewählt aus der Gruppe bestehend aus Wasserstoff, C₁-C₂₀ Alkyl, C₁-C₁₀ Haloalkyl, Halogeno, und wobei R¹ und R² auch miteinander verbrückt sein können,
worin
R' für einen mindestens ein N-Atom enthaltenden aromatischen Rest mit 4 bis 13 C-Atomen steht, der in 2-Stellung zu dem mindestens einen aromatischen N-Atom, an das Stickstoffatom gemäß Formel I gebunden ist, wobei bis zu drei der aromatischen C-Atome durch mindestens ein weiteres Heteroatom, unabhängig voneinander ausgewählt aus der Gruppe bestehend aus N, O und S, substituiert sein können,
worin
R" für Trimethylsilyl oder für einen aromatischen Rest steht, wobei es sich bei R" um einen Rest ausgwählt aus der Gruppe bestehend aus 3-Methylpyridyl, 4-Methylpyridyl, 6-Methylpyridyl, 4,6-Dimethylpyridyl, 6-Methoxypyridyl, Pyridyl, Pyrimidyl, Pyrazinyl, 4-Methylchinolinyl, Py₂(o-P), Phenyl, Naphthyl, Fluorenyl und Trimethylsilyl handelt, wobei die N-haltigen aromatischen Reste in 2-Stellung an das N-Atom gemäß Formel I gebunden sind.
wobei die für
R' und R" aufgeführten Reste desweiteren unabhängig voneinander neben Wasserstoffatomen jeweils mindestens einen Substituenten aufweisen können, die unabhängig voneinander ausgewählt sein können aus der Gruppe bestehend aus C₁- bis C₈-Alkyl, O-Alkyl(C₁-C₈), Aryl, Fluor, CF₃, NHAryl, NAryl₂, wobei Aryl einen Aromaten mit 5 bis 14 Ringkohlenstoffatomen darstellt, wobei ein oder mehrere Ringkohlenstoffatome durch Stickstoff-, Sauerstoff- und/oder Schwefelatome ersetzt sein können und die Alkylreste verzweigt, unverzweigt und/oder zyklisch sein können.

2. Amine gemäß Anspruch 1, wobei es sich bei R¹ und R² unabhängig voneinander um einen Rest ausgewählt aus der Gruppe bestehend aus Phenyl, Cyclohexyl, Alkyl, 2-Alkylphenyl, 3-Alkylphenyl, 4-Alkylphenyl, 2,6-Dialkylphenyl, 3,5-Dialkylphenyl, 3,4,5-Trialkylphenyl, 2-Alkoxyphenyl, 3-Alkoxyphenyl, 4-Alkoxyphenyl, 2,6-Dialkoxyphenyl, 3,5-Dialkoxyphenyl, 3,4,5-Trialkoxyphenyl, 3,5-Dialkyl-4-Alkoxyphenyl handelt, wobei die vorgenannten Alkyl und Alkoxygruppen jeweils vorzugsweise unabhängig voneinander 1 bis 6 Kohlenstoffatome enthalten.

3. Amine gemäß Anspruch 1, wobei es sich bei R¹ und R² unabhängig voneinander um einen Rest ausgewählt aus der Gruppe bestehend aus Phenyl, tert. Butyl oder Cyclohexyl handelt.

4. Amine gemäß Anspruch 1, wobei die Reste R¹ und R² identisch sind.

5. Amine gemäß Anspruch 1, wobei es sich bei R' um einen Rest ausgewählt aus der Gruppe bestehend aus 3-Methylpyridyl, 4-Methylpyridyl, 6-Methylpyridyl, 4,6-Dimethylpyridyl, 6-Methoxypyridyl, Pyridyl, Pyrimidyl, Pyrazinyl, 4-Methylchinolinyl handelt, wobei R' in 2-Stellung an das N-Atom gemäß Formel I gebunden ist.

6. Amine gemäß Anspruch 1, wobei es sich bei dem N-haltigen aromatischen Rest mit 4 bis 13 C-Atomen um Pyrimid-2-yl, 2-Pyridyl, Pyrazin-2-yl oder Chinolin-2-yl handelt.

7. Amine gemäß Anspruch 1, wobei es sich bei den Substituenten für R' und R" um einen Rest unabhängig voneinander ausgewählt aus der Gruppe bestehend aus C₁-C₈-Alkyl, O-Alkyl(C₁-C₈), Aryl, Fluor, CF₃ handelt.

8. Amine gemäß Anspruch 1, wobei es sich bei den Substituenten für R¹, R² , R' und R" unabhängig voneinander um einen Rest ausgewählt aus der Gruppe bestehend aus C₁-C₆ Alkyl, O-C₁-C₆ Alkyl handelt.

9. Verfahren zur Herstellung eines Amins gemäß Anspruch 1, wobei eine Verbindung der Formel NHR'R" in Gegenwart einer starken Base mit einer Verbindung der Formel R¹(R²)PHal umgesetzt wird,
wobei R¹, R² , R' und R" dieselbe Bedeutung haben wie in Anspruch 1 angegeben und Hal für Chlor oder Brom steht.

10. Verfahren nach Anspruch 9, wobei es sich bei der starken Base um ein metallorganisches Reagens handelt.

11. Übergangsmetallkomplex-Verbindung, umfassend mindestens einen Liganden gemäß Anspruch 1 und mindestens ein Übergangsmetall aus der VIII. Nebengruppe des Periodensystems.

12. Komplex-Verbindung nach Anspruch 11, wobei es sich bei dem Übergangsmetall um Palladium, Nickel, Platin, Rhodium, Iridium, Ruthenium und/oder Cobalt handelt.

13. Komplex-Verbindung nach Anspruch 11, wobei es sich bei dem Übergangsmetall um Palladium oder Nickel handelt.

14. Komplex-Verbindung nach Anspruch 11, wobei das Übergangsmetallatom Teil eines 5-gliedrigen Rings ist.

15. Verfahren zur Herstellung eines Komplexes gemäß Anspruch 11, wobei ein Ligand gemäß Anspruch 1 mit einem Komplex und/oder Salz eines Übergangsmetalls der VIII. Nebengruppe des Periodensystems umgesetzt wird.

16. Verwendung eines Liganden gemäß Anspruch 1 als Katalysator in Kombination mit Übergangskomplexen oder Übergangsmetallsalzen der VIII: Nebengruppe des Periodensystems der Elemente, zur Aktivierung von Aryl- oder Vinylhalogeniden für die Herstellung von Dienen oder arylierten Olefinen gemäß der Heck-Reaktion, Biarylen gemäß der Suzuki-Reaktion, α-Arylketonen und/oder Aminen aus Arylhalogeniden oder Vinylhalogeniden oder für Carbonylierungen von Arylhalogeniden, Alkylierungen mit Alkinen gemäß der Sonogashira-Kupplung und Kreuzkupplungen mit metallorganischen Reagenzien oder für die Herstellung von Arylolefinen, Dienen, Diarylen, Benzoesäurederivaten, Acrylsäurederivaten, Arylalkanen, Alkinen und/oder Aminen.

17. Verwendung nach Anspruch 16, **dadurch gekennzeichnet, daß** die Reaktion bei Temperaturen von 0 bis 200 °C durchgeführt wird.

18. Verwendung nach Anspruch 16, **dadurch gekennzeichnet, daß** bei der katalytischen Anwendung der Phosphanligand im Verhältnis Übergangsmetall zu Ligand 1:1 bis 1:1000 eingesetzt wird.

19. Verwendung nach Anspruch 18, **dadurch gekennzeichnet, daß** das Verhältnis Übergangsmetall zu Ligand 1:1 bis 1:100 beträgt.

20. Verwendung nach Anspruch 16, wobei die Übergangsmetallkonzentration zwischen 5 Mol-% und 0,01 Mol-% liegt.

21. Verwendung nach Anspruch 20, wobei die Übergangsmetallkonzentration zwischen 1 Mol-% und 0,01 Mol-% liegt.

22. Amin ausgewählt aus der Gruppe bestehend aus N-(4-methylpyrid-2-yl)-N-(pyrimid-2-yl)amin, N-(4,6-dimethylpyrid-2-yl)-N-(6-methoxypyrid-2-yl)amin, N,N-bis(pyrazinyl)amin, N,N,N'-tris(pyrid-2-yl)-o-phenylendiamin.

## Revendications

1. Composés aromatiques contenant de l'azote et des amines fonctionnalisées P de formule générale
R¹(R²)P-N(R')R'' (I)
dans laquelle
R¹ et R² représentent indépendamment l'un de l'autre un radical choisi dans le groupe constitué d'un alkyle en C₁-C₂₄, d'un cycloalkyle en C₃-C₈ et de radicaux aromatiques comprenant 5 à 14 atomes C, les radicaux alkyle et cycloalkyle pouvant être ramifiés ou non ramifiées,
les radicaux susmentionnés pouvant être chacun substitués une ou plusieurs fois par des radicaux choisis indépendamment les uns des autres dans le groupe constitué d'un hydrogène, d'un alkyle en C₁-C₂₀, d'un halogénoalkyle en C₁-C₁₀ et d'un halogène, et R¹ et R² pouvant être également pontés l'un avec l'autre,
dans laquelle
R' représente un radical aromatique comprenant 4 à 13 atomes C contenant au moins un atome N et lié en deuxième position par rapport audit au moins un atome aromatique N à l'atome d'azote selon la formule I, jusqu'à trois des atomes aromatiques C pouvant être substitués par au moins un hétéroatome supplémentaire choisi indépendamment dans le groupe constitué de N, O et S,
dans laquelle
R'' représente un triméthylsilyle ou un radical aromatique, R'' étant un radical choisi dans le groupe constitué de 3-méthylpyridyle, 4-méthylpyridyle, 6-méthylpyridyle, 4,6-diméthylpyridyle, 6-méthoxypyridyle, pyridyle, pyrimidyle, pyrazinyle, 4-méthylquinoléinyle, Py₂(o-P), phényle, naphtyle, fluorényle et triméthylsilyle, les radicaux aromatiques contenant N étant liés en deuxième position à l'atome N selon la formule I,
les radicaux indiqués pour R' et R" pouvant en outre chacun présenter indépendamment l'un de l'autre, en plus d'atomes d'hydrogène, au moins un substituant choisi indépendamment dans le groupe constitué de alkyle en C₁-C₈, O-(alkyle en C₁-C₈), aryle, fluor, CF₃, NH-aryle et N-aryle₂, le radical aryle représentant un composé aromatique comprenant 5 à 14 atomes de carbone cycliques, un ou plusieurs des atomes de carbone cycliques pouvant être substitués par des atomes d'azote, d'oxygène et/ou de soufre et les radicaux alkyle pouvant être ramifiés, non ramifiés et/ou cycliques.

2. Amines selon la revendication 1, dans lesquelles R¹ et R² sont indépendamment l'un de l'autre un radical choisi dans le groupe constitué de phényle, cyclohexyle, alkyle, 2-alkylphényle, 3-alkylphényle, 4-alkylphényle, 2,6-dialkylphényle, 3,5-dialkylphényle, 3,4,5-trialkylphényle, 2-alcoxyphényle, 3-alcoxyphényle, 4-alcoxyphényle, 2,6-dialcoxyphényle, 3,5-dialcoxyphényle, 3,4,5-trialcoxyphényle, 3,5-dialkyl-4-alcoxyphényle, les groupements alkyle et alcoxy susmentionnés comprenant chacun de préférence indépendamment l'un de l'autre 1 à 6 atomes de carbone.

3. Amines selon la revendication 1, dans lesquelles R¹ et R² sont indépendamment l'un de l'autre un radical choisi dans le groupe constitué d'un phényle, d'un tert-butyle ou d'un cyclohexyle.

4. Amines selon la revendication 1, dans lesquelles les radicaux R¹ et R² sont identiques.

5. Amines selon la revendication 1, dans lesquelles R' est un radical choisi dans le groupe constitué de 3-méthylpyridyle, 4-méthylpyridyle, 6-méthylpyridyle, 4,6-diméthylpyridyle, 6-méthoxypyridyle, pyridyle, pyrimidyle, pyrazinyle, 4-méthylquinoléinyle, R' étant lié en deuxième position à l'atome N selon la formule I.

6. Amines selon la revendication 1, dans lesquelles le radical aromatique comprenant 4 à 13 atomes C contenant N est pyrimid-2-yle, 2-pyridyle, pyrazin-2-yle ou quinoléin-2-yle.

7. Amines selon la revendication 1, dans lesquelles les substituants pour R' et R" sont indépendamment les uns des autres un radical choisi dans le groupe constitué de alkyle en C₁-C₈, O-(alkyle en C₁-C₈), aryle, fluor et CF₃.

8. Amines selon la revendication 1, dans lesquelles les substituants pour R¹, R², R' et R" sont indépendamment les uns des autres un radical choisi dans le groupe constitué de alkyle en C₁-C₆ et O-(alkyle en C₁-C₆).

9. Procédé de préparation d'une amine selon la revendication 1, dans lequel un composé de formule NHR'R" est transformé en présence d'une base forte avec un composé de formule R¹(R²)PHal, R¹, R², R' et R'' ayant la même signification qu'indiqué à la revendication 1 et Hal représentant le chlore ou le brome.

10. Procédé selon la revendication 9, dans lequel la base forte est un réactif organométallique.

11. Composé complexe de métal de transition comprenant au moins un ligand selon la revendication 1 et au moins un métal de transition du sous-groupe VIII du système périodique.

12. Composé complexe selon la revendication 11, dans lequel le métal de transition est le palladium, le nickel, le platine, le rhodium, l'iridium, le ruthénium et/ou le cobalt.

13. Composé complexe selon la revendication 11, dans lequel le métal de transition est le palladium ou le nickel.

14. Composé complexe selon la revendication 11, dans lequel l'atome du métal de transition fait partie d'un cycle à 5 chaînons.

15. Procédé de préparation d'un complexe selon la revendication 11, dans lequel un ligand selon la revendication 1 est transformé avec un complexe et/ou un sel d'un métal de transition du sous-groupe VIII du système périodique.

16. Utilisation d'un ligand selon la revendication 1 comme catalyseur en combinaison avec des complexes de transition ou des sels de métaux de transition du sous-groupe VIII du système périodique des éléments en vue d'activer des halogénures d'aryle ou de vinyle pour la préparation de diènes ou d'oléfines arylées selon la réaction de Heck, de biaryles selon la réaction de Suzuki, d'α-arylcétones et/ou d'amines à partir d'halogénures d'aryle ou d'halogénures de vinyle ou pour des carbonylations d'halogénures d'aryle, des alkylations avec des alcynes selon le couplage de Sonogashira et des couplages croisés avec des réactifs organométalliques ou pour la préparation d'aryloléfines, de diènes, de diaryles, de dérivés de l'acide benzoïque, de dérivés de l'acide acrylique, d'arylalcanes, d'alcynes et/ou d'amines.

17. Utilisation selon la revendication 16, **caractérisée en ce que** la réaction est réalisée à des températures de 0 à 200°C.

18. Utilisation selon la revendication 16, **caractérisée en ce que** lors d'une application catalytique le ligand de phosphane est utilisé dans un rapport du métal de transition au ligand de 1 : 1 à 1 : 1000.

19. Utilisation selon la revendication 18, **caractérisée en ce que** le rapport du métal de transition au ligand est de 1 : 1 à 1 : 1 100.

20. Utilisation selon la revendication 16, dans laquelle la concentration en métal de transition se situe entre 5% en mole et 0,01% en mole.

21. Utilisation selon la revendication 20, dans laquelle la concentration en métal de transition se situe entre 1% en mole et 0,01% en mole.

22. Amine choisie dans le groupe constitué de N-(4-méthylpyrid-2-yl)-N-(pyrimid-2-yl)amine, N-(4,6-diméthylpyrid-2-yl)-N-(6-méthoxypyrid-2-yl)amine, N,N-bis(pyrazinyl)amine et N,N,N'-tris(pyrid-2-yl)-o-phénylènediamine.

## Claims

1. P-functionalized amines of N-containing aromatics having the general formula
R¹(R²)P-N(R')R" (I)
where
R¹ and R² are each, independently of one another, a radical selected from the group consisting of C₁-C₂₄-alkyl, C₃-C₈-cycloalkyl and aromatic radicals having from 5 to 14 carbon atoms, with the alkyl and cycloalkyl radicals being able to be branched or unbranched,
and
with the abovementioned radicals themselves each being able to be substituted by one or more radicals selected independently from the group consisting of hydrogen, C₁-C₂₀-alkyl, C₁-C₁₀-haloalkyl, halo, and R¹ and R² also being able to be joined by a bridge,
and
R' is an aromatic radical which contains at least one N atom and has from 4 to 13 carbon atoms and is bound in the 2 position relative to the at least one aromatic N atom to the nitrogen atom in the formula I, with up to three of the aromatic carbon atoms being able to be substituted by at least one further heteroatom selected independently from the group consisting of N, O and S,
and
R" is trimethylsilyl or an aromatic radical and is selected from the group consisting of 3-methylpyridyl, 4-methylpyridyl, 6-methylpyridyl, 4,6-dimethylpyridyl, 6-methoxypyridyl, pyridyl, pyrimidyl, pyrazinyl, 4-methylquinolinyl, Py₂(o-P), phenyl, naphthyl, fluorenyl, and trimethylsilyl, with the N-containing aromatic radicals being bound in the 2 position to the N atom in the formula I and with the
radicals mentioned for R' and R" also being able to bear, independently of one another, not only hydrogen atoms but in each case also at least one substituent which can be selected independently from the group consisting of C₁-C₈-alkyl, O-alkyl(C₁-C₈), aryl, fluorine, CF₃, NHaryl, Naryl₂, with aryl being an aromatic having from 5 to 14 ring carbons and one or more ring carbons being able to be replaced by nitrogen, oxygen and/or sulphur atoms and the alkyl radicals being able to be branched, unbranched and/or cyclic.

2. Amines according to Claim 1, wherein R¹ and R² are each, independently of one another, a radical selected from the group consisting of phenyl, cyclohexyl, alkyl, 2-alkylphenyl, 3-alkylphenyl, 4-alkylphenyl, 2,6-dialkylphenyl, 3,5-dialkylphenyl, 3,4,5-trialkylphenyl, 2-alkoxyphenyl, 3-alkoxyphenyl, 4-alkoxyphenyl, 2,6-dialkoxyphenyl, 3,5-dialkoxyphenyl, 3,4,5-trialkoxyphenyl, 3,5-dialkyl-4-alkoxyphenyl, with the abovementioned alkyl and alkoxy groups each preferably containing, independently of one another, from 1 to 6 carbon atoms.

3. Amines according to Claim 1, wherein R¹ and R² are each, independently of one another, a radical selected from the group consisting of phenyl, tert-butyl and cyclohexyl.

4. Amines according to Claim 1, wherein the radicals R¹ and R² are identical.

5. Amines according to Claim 1, wherein R' is a radical selected from the group consisting of 3-methylpyridyl, 4-methylpyridyl, 6-methylpyridyl, 4,6-dimethylpyridyl, 6-methoxypyridyl, pyridyl, pyrimidyl, pyrazinyl, 4-methylquinolinyl, with R' being bound in the 2 position to the N atom in the formula I.

6. Amines according to Claim 1, wherein the N-containing aromatic radical having from 4 to 13 carbon atoms is pyrimid-2-yl, 2-pyridyl, pyrazin-2-yl or quinolin-2-yl.

7. Amines according to Claim 1, wherein the substituents on R' and R" are each a radical selected independently from the group consisting of C₁-C₈-alkyl, O-alkyl(C₁-C₈), aryl, fluorine, CF₃.

8. Amines according to Claim 1, wherein the substituents on R¹, R², R' and R" are each, independently of one another, a radical selected from the group consisting of C₁-C₆-alkyl, O-C₁-C₆₋alkyl.

9. Process for preparing an amine according to Claim 1, wherein a compound of the formula NHR'R" is reacted with a compound of the formula R¹(R²)PHal in the presence of a strong base, where R¹, R², R' and R" are as defined in Claim 1 and Hal is chlorine or bromine.

10. Process according to Claim 9, wherein the strong base is an organometallic reagent.

11. Transition metal complex comprising at least one ligand according to Claim 1 and at least one transition metal from transition group VIII of the Periodic Table.

12. Complex according to Claim 11, wherein the transition metal is palladium, nickel, platinum, rhodium, iridium, ruthenium and/or cobalt.

13. Complex according to Claim 11, wherein the transition metal is palladium or nickel.

14. Complex according to Claim 11, wherein the transition metal atom is part of a 5-membered ring.

15. Process for preparing a complex according to Claim 11, wherein a ligand according to Claim 1 is reacted with a complex and/or salt of a transition metal of transition group VIII of the Periodic Table.

16. Use of a ligand according to Claim 1 as catalyst in combination with transition metal complexes or transition metal salts of transition group VIII of the Periodic Table of the Elements for activating aryl or vinyl halides for the preparation of dienes or arylated olefins by the Heck reaction, biaryls by the Suzuki reaction, α-aryl ketones and/or amines from aryl halides or vinyl halides or for carbonylations of aryl halides, alkylations by means of alkynes by Sonogashira coupling and cross couplings with organometallic reagents or for the preparation of arylolefins, dienes, diaryls, benzoic acid derivatives, acrylic acid derivatives, arylalkanes, alkynes and/or amines.

17. Use according to Claim 16, **characterized in that** the reaction is carried out at temperatures of from 0 to 200°C.

18. Use according to Claim 16, **characterized in that**, in the catalytic application, the phospane ligand is used in a ratio of transition metal to ligand of from 1:1 to 1:1000.

19. Use according to Claim 18, **characterized in that** the ratio of transition metal ligand is from 1:1 to 1:100.

20. Use according to Claim 16, wherein the transition metal concentration is in the range from 5 mol% to 0.01 mol%.

21. Use according to Claim 20, wherein the transition metal concentration is in the range from 1 mol% to 0.01 mol%.

22. Amine selected from the group consisting of N-(4-methylpyrid-2-yl)-N-(pyrimid-2-yl)amine, N-(4,6-dimethylpyrid-2-yl)-N-(6-methoxypyrid-2-yl)amine, N,N-bis(pyrazinyl)amine, N,N,N'-tris(pyrid-2-yl)-o-phenylenediamine.
